# EUROPEAN PATENT APPLICATION

(11) **EP 1 637 594 A2**
(43) Date of publication of application: **22.03.2006**
(21) Application number: 02080694.9
(22) Date of filing: 02.04.2002
(51) Int. Cl.: C12N 9/02

(54) **Methods of purification of Cytochrome P450 proteins**

(30) Priority: 02.04.2001 GB 0108212; 02.04.2001 GB 0108214; 23.07.2001 US 306873 P; 23.07.2001 US 306874 P
(71) Applicant: Astex Technology Limited, Cambridge, Cambridgeshire CB4 0WE (GB)
(72) Inventor: Ward, Alison, Astex Therapeutics Limited, Cambridge CB4 0QA (GB); Cosme, Jose, Astex Therapeutics Limited, Cambridge CB4 0QA (GB); Williams, Pamela, Astex Therapeutics Limited, Cambridge CB4 0QA (GB); Hamilton, Bruce, Astex Therapeutics Limited, Cambridge CB4 0QA (GB); Vuillard, Laurent, Astex Therapeutics Limited, Cambridge CB4 0QA (GB)
(74) Representative: Brasnett, Adrian Hugh

(57) **Abstract**

The invention provides a method for the purification of cytochrome P450 molecules, the method comprising expressing in a host cell culture a cytochrome P450 molecule; recovering said cells from said culture and suspending said cells in a high salt buffer; lysing said cells and removing cell debris to provide a high-salt lysate; adding to said lysate a detergent to provide a high-salt-detergent lysate; and recovering said P450 from said lysate. The method provides yields of P450 proteins suitable for crystallization.

## Description

The present invention relates to methods for preparing cytochrome P450 molecules, particularly in a form suitable for crystallisation.

Cytochrome P450s are a very large and complex gene superfamily of hemeproteins that metabolise physiologically important compounds in many species of microorganisms, plants and animals. Cytochrome P450s are important in the oxidative, peroxidative and reductive metabolism of numerous and diverse endogenous compounds such as steroids, bile, fatty acids, prostaglandines, leukotrienes, retinoids and lipid. Many of these enzymes also metabolise a wide range of xenobiotics including drugs, environmental compounds and pollutants.

Mammalian cytochrome P450s are 50-55 kDa heme-thiolate enzymes that are found in either the mitochondrial inner membrane (type I) or in the endoplasmic reticulum network of the cell (type II). The type II or microsomal enzymes are integral membrane proteins anchored to the membrane by an N-terminal transmembrane spanning α-helix. The bulk of the enzyme faces the cytoplasmic surface of the lipid bilayer as opposed to the lumen. For activity, cytochrome P450s require other membranous enzymatic components including the flavoprotein NADPH-cytochrome P450 oxidoreductase and, in some cases, cytochrome b5. A single cytochrome P450 oxidoreductase supports the activity of all the mammalian microsomal enzymes by interacting directly with the P450s and transferring the required two electrons from NADPH. Cytochrome b5 is necessary for increasing electron transfer for certain P450 isoforms and specific substrates. Cytochrome P450s are able to incorporate one of the two oxygen atoms of an O₂ molecule into a broad variety of substrates with concomitant reduction of the other oxygen atom by two electrons to H₂O. Cytochrome P450 are known to catalyze hydroxylations, epoxidation, N-, S-, and O-dealkylations, N-oxidations, sulfoxidations, dehalogenations, and other reactions.

The genes of the P450 superfamily have been categorised by Nelson et al (Pharmacogenetics, 6;1-42, 1996), the disclosure of which is incorporated herein by reference, who proposed a systematic nomeclature for the family members. This nomenclature is used widely in the art, and is adopted herein (the prefix of "CYP" is omitted when referring to subfamily groups). Nelson et al provide cross-references to database sequence entries for P450 sequences.

*Homo sapiens* has 17 cytochrome P450 gene families and 42 subfamilies that total more than 50 sequenced isoforms. Cytochrome P450s from families 1, 2 and 3 constitute the major pathways for drug metabolism. Many drugs rely on hepatic metabolism by cytochrome P450s for clearance from the circulation and for pharmacological inactivation. Conversely, some drugs have to be converted in the body to their pharmacologically active metabolites by P450s. It is estimated that 50% of all known drugs are modified by P450s and many promising lead compounds are terminated in the development phase due to their interaction with a cytochrome P450. One of the greatest problems in drug discovery is the prediction of the role of cytochrome P450s on the metabolism or modification of drug leads. Early detection of metabolic problems associated with a chemical lead series is a paramount importance for the drug industry. Obtaining crystal structures of the main human drug metabolising cytochrome P450s would be highly valuable for drug design, as this would provide detailed information on how P450 enzymes recognise drug molecules and the mode of drug binding. This in turn would allow drug companies to develop strategies to modify metabolic clearance and decrease the attrition rates of compounds in development.

To date eight cytochrome P450 structures have been solved by X-ray crystallography and are available in the public domain. Five structures correspond to bacterial cytochrome P450s: P450cam (CYP101 Poulos et *al.,* 1985, J. *Biol. Chem.,* 260, 16122), the hemeprotein domain of P450BM3 (CYP102 , Ravichandran et *al.,* 1993, *Science,* 261, 731), P450terp (CYP108, Hasemann et *al.,* 1994, J. *Mol. Biol.* 236, 1169), P450eryF (CYP107A1, Cupp-Vickery and Poulos, 1995, *Nature* Struct. Biol. 2, 144), and P450 14α-sterol demethylase (CYP51, Podust et *al.,* 2001, *Proc. Natl. Acad. Sci. USA,* 98, 3068). The structure of cytochrome P450nor was obtained from the denitrifying fungus Fusarium oxysporum (Shimizu et *al.* 2000, J. *Inorg. Biochem.* 81, 191). Recently the crystal structure of a thermophilic cytochrome P450 (CYP119) from Archaeon sulfolobus solfataricus has been reported (Yano et *al.,* 2000, J. *Biol. Chem.* 275, 31086-31092). The eighth structure is the rabbit 2C5 isoform, the first structure of a mammalian cytochrome P450 (Williams et *al.* 2000, *Mol. Cell.* 5, 121-131). All of the cytochrome P450s, whose structures have been solved, were expressed in *E*. *coli.* The reason to date why, the mammalian cytochrome P450s have been particularly difficult to be crystallise, compared to their bacterial counterparts, resides in the nature of these proteins. The bacterial cytochrome P450s are soluble whereas the mammalian P450s are integral membrane proteins. The mammalian cytochrome P450s are inserted in the membrane of the endoplasmic reticulum by a short, highly hydrophobic N-terminal segment that acts as a non cleavable signal sequence for insertion into the membrane. The remainder of the mammalian cytochrome P450 protein is a globular structure that protrudes into the cytoplasmic space.

Most of the mammalian cytochrome P450s are located in the liver, but other organs and tissues have high concentrations of certain cytochrome P450s, including the intestinal wall, lung, kidney, adrenal cortex and nasal epithelium. While a number of cytochrome P450 enzymes have been successfully isolated from mammalian microsomes, purification from tissue is not trivial. Purification is complicated by low cytochrome P450 availability in human tissues, the limited yields, difficulties to isolate highly related isoforms that share a high amino acid identity, the hydrophobic nature of these proteins, the use of detergent and, at times, the loss of activity during the purification process. The method of purification from tissues requires the preparation of the microsomal membranes, the extraction of the membrane associated cytochrome P450s with detergent, successive purification steps and the removal of the detergent in the final purification. The cytochrome P450s that have been isolated following this procedure have exhibited a high tendency to aggregate, low solubility and poor monodispersity, features not conducive to crystallization.

Structural studies on mammalian cytochrome P450s may require the combination of heterologous expression systems that allow expression of single cytochrome P450s at high concentration, and modifications in their sequences to improve the solubility and the behaviour of these proteins in solution. Mammalian cytochrome P450s are now routinely expressed as recombinant proteins in many different systems, including mammalian cell culture, yeast, baculovirus and bacteria (refer to Methods in Enzymology, Vol. 206, Academic Press, 1991). The latter system has been employed successfully for the expression of a number of microsomal P450 enzymes, in large amounts and at low costs for biophysical or structural studies (Barnes et *al.,* 1991, *Proc. Natl. acad. Sci. USA* 88 , 5597-5601). Expression of several full-length mammalian cytochrome P450s in E. *coli* is now well documented in the literature (Fisher et *al.* 1992, *FASEB J.* 6, 759-764; Richardson et al. 1995, *Arch Biochem. Biophys.* 323, 87-96, Gillam et *al.* 1995, *Arch. Biochem. Biophys.* 317, 374-384). Modification of a few residues at the N-terminus of the protein has been described as advantageous to optimise expression in *E*. *coli.* The change of the second codon to an alanine residue is commonly used. However, the physical properties of these full-length cytochrome P450s are very similar to those described for the cytochrome P450s isolated from mammalian tissues, and therefore, the resulting proteins may not be amenable to structural studies.

It is now well recognized that the deletion of the N-terminal membrane anchor region in conjunction with the high level of expression achieved in heterologous systems could provide the means to obtain preparative amounts of soluble mammalian cytochrome P450s for X-ray crystallography. This is based in the assumption that by eliminating the N-terminal hydrophobic signal sequence, a functional cytochrome P450 form lacking its microsomal membrane anchor is produced. Such proteins would be expected to be less hydrophobic, probably even soluble, and presumably more amenable to X-ray crystallography.

Several attempts to produce soluble mammalian cytochrome P450s by removing the N-terminus have been reported in the literature by different groups. Similar N-terminal deletions of cytochrome P450s have been used in each case, though the methods of purification that have been applied and the results obtained differ greatly. There is thus a need for an improved and more generally applicable method of purification.

Cytochrome P450 N-terminal deletion has been successfully applied by Johnson's group to solve the X-ray crystallographic structure of the first mammalian P450 (Williams et *al.* 2000, *Mol. Cell.* 5, 121-131). This group (Wachenfelt et *al.,* 1997, *Arch. Biochem. Biophys.* 339, 107-114; Cosme et *al.,* 2000, J. *Biol. Chem.* 275, 2545-2553) have expressed the rabbit 2C3 and 2C5 isoforms in E. *coli* with residues 3-21 at the N-terminus deleted. The results indicate that the truncated proteins exhibit a salt-dependent association with the cell membrane suggesting that the removal of the membrane spanning domain converts these intrinsic membrane proteins to peripherical membrane proteins that could be purified without the use of detergent. The resulting purified proteins were predominantly dimers and tetramers. Difficulties in the crystallisation of the rabbit cytochrome P450 2C5 were overcome by the combination of the removal of the membrane spanning sequence and engineering in a region that is believed to be involved in the membrane association.

Kempf et *al.* (Kempf et *al.,* 1995, *Arch. Biochem. Biophys.,* 321, 277-288) have analyzed the expression in E. *coli* of a series of constructs of the human cytochrome P450 2D6 where the N-terminal 25 amino acids were replaced with a hexahistidine following the N-terminal Met codon. The protein was solubilized from the membrane in high salt and then purified in presence, or absence, of the detergent C₁₂E₉ using Ni²⁺⁻chelate affinity chromatography followed by purification using DEAE-Sephacel and Hydroxylapatite resin. Their results have shown that in the absence of detergents, the protein exhibits a strong tendency to aggregate into multimeric states. Greater than 50 % of the protein is highly aggregated. The protein could be dissociated to a mononer if the nonionic detergent NP40 was used during the purification and kept in the sample. Three columns were required to achieve a high purity and final yield of 20%.

Gillam *et al,* (Gillam et *al.,* 1995, *Arch. Biochem. Biophys.* 319, 540-550), have examined a series of N-terminal modified constructs of human cytochrome P450 2D6 for expression in *E*. *coli.* Although several of these produced some cytochrome P450 2D6, there was considerable variation in the expression rate. The best construct involved the removal of the N-terminal hydrophobic region. The expressed cytochrome P450s were first, extracted from membranes in presence of detergent Triton X114 and then purified using a flavodoxin affinity column and a hydroxylapatite in presence of detergent. The detergent was eliminated in the final preparation by dialysis or by extensive washing. Their results have shown that even deleted of their membrane anchoring sequence, the proteins still bind the membrane and need to be extracted with detergents. The overall recovery of cytochrome P450 2D6 is less than 20%. No data on the aggregation state of the protein was presented.

Pernecky et *al.* (Pernecky et *al. Proc. Natl. Acad. Sci. USA,* 1993, 90, 2651-2655) have expressed the rabbit N-truncated forms of cytochrome P450 2B4 and 2E1 and several chimeras in *E. coli.* The proteins were extracted with 1% n-octyl β D glucopyranoside from the bacterial lysate and successively purified on GSH-Sepharose column and treated with thrombin in order to release the GST tag. Finally, residual detergent was removed from the preparation on a hydroxyapatite column. The results have shown that in absence of detergent, the truncated cytochrome P450 2E1 is still largely aggregated (pentamers) and cytochrome P450 2B4 was a mixture of high-molecular-weight aggregates with an average octomeric size. Addition of detergent was required to convert the protein to a monomeric form.

Larson et *al.* (Larson et *al.,* 1991, J. *Biol. Chem,* 266, 7321-7324) found that rabbit cytochrome P450 2E1 expressed in *E*. *coli* without amino acids 3-29, like the full-length cytochrome P450, was predominantly in the membrane fraction. The inability to liberate the truncated cytochrome P450 from membranes with 0.1M Na₂CO₃, at pH 11, provides evidence for an integral association of the shortened cytochrome P450 2E1 with the membrane. The protein was partially purified after it solubilization from the *E. coli* membrane using n-octyl β D glucopyranoside as detergent.

Saraga et *al.* (Saraga et al., 1993, *Arch. Biochem. Biophys.* 304, 272-278) have expressed a truncated form of the bovine microsomal 17β-hydroxylase cytochrome P450 (P450c17) lacking its N-terminal hydrophobic signal anchor sequence (residues 2-17). The results indicate that the truncated cytochrome P450 is primarily associated with the membrane. No information on this protein was presented.

### Disclosure of the Invention.

Despite the efforts such as those of the prior art cited above, there is still a need to develop methods for the purification of P450s from recombinant host cells in a reliable manner, and in yields which allow crystals of the proteins to be formed in sufficient quantity and quality to be amenable for X-ray crystallographic studies and crystallographic screening of small molecules bound to P450 proteins. Purified P450 proteins are also useful for many other purposes, such as NMR studies and high throughput screening methods to discover drugs or analyse the interaction of drugs with these molecules.

The present invention aims to overcome the difficulties of the prior art by providing for efficient isolation and purification of P450s. In our studies we have found that a problem with the prior art is that P450 proteins tend to aggregate during their isolation and purification. We believe that one reason for this, which has not previously been identified, is the perceived need in the art to resuspend host cells expressing P450s in a buffer with low ionic strength prior to lysis.

In addition, the prior art also teaches that after lysis it is necessary to recover the membrane fraction (usually by high speed ultracentrifugation of the cell lysate) of the host cells before bringing the cells into contact with a detergent in order to remove the P450s from the membrane fraction.

These steps reduce the yield of P450 recovered from the cell lysate. We have now found a means to recover P450 from a cell lysate without the need to recover a separate membrane fraction. The process uses a buffer with a high ionic strength (i.e. a high concentration of salt) at an earlier stage in the recovery process, and provides for a high recovery of protein in a non-aggregated state.

Thus in a first aspect, the invention provides a method for the purification of P450, wherein said method comprises:
(a) expressing in a host cell culture a cytochrome P450 molecule;
(b) recovering said cells from said culture and suspending said cells in a salt buffer having a conductivity of from 12 to 110 mS/cm;
(c) lysing said cells and removing cell debris to provide a high-salt lysate;
(d) adding to said lysate a detergent to provide a high-salt-detergent lysate; and
(e) recovering said P450 from said lysate.

Preferably the salt buffer has a salt concentration of from 200 to 1000 mM.

Preferably the detergent is added at 0.015 to 1.2% v/v.

Alternatively, the detergent may be added prior to removal of the cell debris in step (c), though this is not preferred.

Most preferably, the salt buffer has a salt concentration of from 200 to 1000 mM and the detergent is added at 0.015 to 1.2% v/v.

The recovery step generally involves affinity purification of the P450 from the high-salt-detergent lysate, since the presence of the high salt rules out the alternative of an immediate ionic exchange purification step.

However, once the P450 has been purified by affinity purification (e.g. chromatography), the salt needs to be removed in order to allow additional purification of the product so that crystallisation can be performed. In the prior art, salt removal is typically performed by dialysis. However, we have found that this process, which removes salt gradually over several hours (typically a 12 - 24 hour period), causes aggregation and denaturation of the P450s and thus is undesirable. We have found that rapid desalting alleviates this problem to a significant degree.

Thus in a further aspect, step (e) above may be performed by:
(e(i)) binding said P450 to an affinity support;
(e(ii)) rinsing said support in a high-salt-detergent wash;
(e(iii)) removing said P450 in a high-salt-detergent buffer to provide a P450-high-salt-detergent preparation; and
(f) rapidly desalting the preparation to provide a P450-low-salt preparation.

Preferably step (f) is performed by removing salt from said preparation by size-exclusion chromatography or by other methods providing desalting over an equivalent timescale.

The above steps e(i)-(iii) maintain the P450 in a high-salt and detergent buffer throughout the initial stages of the purification process, which aids the recovery of the P450.

The preparation may be subject to additional purification and cleaning procedures, such as cation exchange chromatography, optionally followed by further size-exclusion chromatography to obtain a more purified preparation of protein.

The protein preparation recovered may be crystallised, for example by the hanging drop method or other conventional techniques in the art, and subject to X-ray crystallographic analysis. In a further aspect, the invention thus provides a crystal of a P450 protein molecule, and a method obtaining the crystal structure of a P450 molecule which comprises subjecting said crystal to X-ray diffraction, and analysing the diffraction pattern obtained to determine the 3-dimensional coordinates of the atoms of said P450.

In a particular aspect, the crystal of the invention is P450 2C19 which has cell dimensions of a=158Å, b=158Å, c=212Å, α=90°, β=90°, γ=120°, and a space group P321. In another aspect, the invention provides a crystal of 3A4 having a space group I222 and unit cell size a=77 Å, b=99 Å, c=129 Å, β=90°; or having a space group C2 and unit cell size a=152Å, b=101 Å, c=78Å, α=90°, β=120°, γ=90°.

Those of skill in the art will recognise that the cell dimensions of the crystal may vary by 5%, though preferably by 1-2Å, upon repeat crystallisation, and such variation resides within the spirit and scope of the invention.

### Description of the Drawings.

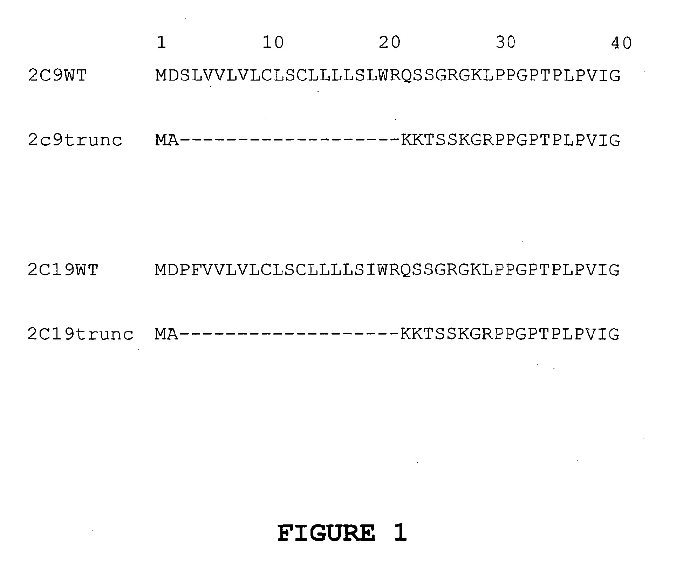

Figure 1 shows the N-terminal sequences of 2C9 and 2C19, together with truncations which remove the membrane-inserting N-terminal region.

### Detailed Description of the Invention.

### Cytochrome P450

We believe that the novel purification process of the invention will be broadly applicable to most cytochrome P450 proteins, since the process is directed to providing at an early stage in purification these proteins in a soluble disaggregated form.

Cytochrome P450 families of interest include the families CYP1, CYP2, CYP3, CYP4, CYP5, CYP6, CYP7A, CYP7B, CYP8, CYP9, CYP10, CYP11, CYP12, CYP13, CYP14, CYP15, CYP16, CYP17, CYP18, CYP19, CYP21, CYP24, CYP26, CYP27, CYP46, CYP51 and CYP52. Of these the families of cytochromes of vertebrates are of particular interest, namely the families CYP1, CYP2, CYP3, CYP4, CYP5, CYP7A, CYP7B, CYP8, CYP11, CYP17, CYP19, CYP21, CYP24, CYP26, CYP27, CYP46 and CYP51.

Of these, the CYP subfamilies include the 1A (particularly 1A1 and 1A2), 1B (particularly 1B1), 2A (particularly 2A6, 2A7, 2A13), 2B (e.g. 2B6), 2C (particularly 2C8, 2C9, 2C18 and 2C19), 2D (particularly 2D6), 2E (particularly 2E1), 2F (particularly 2F1), 2J (particularly 2J2), 3A (particularly 3A4, 3A5 and 3A7), 4A (particularly 4A11, 4A20), 4B (particularly 4B1), 4C, 4D, 4E, 4F (particularly 4F2, 4F3, 4F8, 4F11, 4F12), 5A (particularly 5A1), 7A (particularly 7A1), 7B (particularly 7B1), 8A (particularly 8A1), 8B (particularly 8B1), 11A (particularly 11A1), and 11B (particularly 11B1, 11B2) subfamilies.

Human cytochrome P450 genes are of particular interest, including human genes of the above families and subfamilies. The sequences of the genes are available on a number of public databases, including SwissProt. Human P450s include 1A1 (SwissProt P04798 (all following entries SwissProt unless indicated otherwise)), 1A2 (P05177), 1B1 (Genbank/EMBL U03688), 2A6 (P11509), 2A7 (P20853), 2A13 (Genbank/EMBL U22028), 2B6 (P20813), 2C8 (P10632), 2C9 (P11712), 2C18 (P33260), 2C19 (P33261), 2D6 (P10635), 2E1 (P05181), 2F1 (P24903), 2J2 (Genbank/EMBL U37143), 3A3 (P05184), 3A4 (P08684 or M18907), 3A5 (P20815), 3A7 (Genbank/EMBL NM_000765), 4A20 (Genbank/EMBL AJ131016), 4B1 (P13584), 5A1 (P24557), 4A11 (Genbank/EMBL L04751), 4F2 (Genbank/EMBL U02388), 4F3 (Q08477), 4F8 (Genbank/EMBL AF133298), 4F11 (Genbank/EMBL AF236085), 4F12 (Genbank/EMBL AC004523), 7A (P22680), 7A1 (Genbank/EMBL X56088), 7B1 (Genbank/EMBL AF029403), 8A1 (Genbank/EMBL AF297048), 8B1 (Genbank/EMBL AF090318), 11A1 (P05108), 11B1 (P15538), 11B2 (P19099), 17 (P05093), 19 (P11511), 21 (P04033), 24 (Genbank/EMBL L13286), CYP26 (Genbank/EMBL NM_000783), 27 (Q02318), 46 (Genbank/EMBL NM_006668), and 51 (Genbank/EMBL U23942).

Non-human homologues of the above members are also of interest, i.e. non-human proteins from the same sub-families as those mentioned herein which have a high degree (>70% sequence identity) to the proteins mentioned above. Other mammalian P450s include dog P450s such as 2D15 (Genbank/EMBL D17397) and 3A12 (P24463), and rat such as 3A1 (P04800).

A particular group of proteins of interest are the human CYP2 and CYP3 families and in particular the 2C and 2D subfamilies. There are at least seven subfamilies in the family of CYP2 and these include 2A6, 2A7, 2A13, 2B6, 2C8, 2C9, 2C18 and 2C19, 2D6, 2E1, 2F1 and 2J2. The four human 2C cytochrome P450 molecules, 2C8, 2C9, 2C18 and 2C19 are of particular interest. The 3A4, 3A5 and 3A7 CYPs are also of interest.

In the present invention, it will be understood that reference to cytochromes includes both the full length membrane bound sequences, as well as these proteins in which there is a deletion of the N-terminal segment which inserts the protein into the membrane. Such truncated proteins have been widely generated in the art for the purposes of enhancing purification of P450s. See for example von Wachenfeldt et al, *Archives Biochem. Biophys.* 339, 107-114, 1997. Also included in the invention by reference to cytochromes are allelic variants and mutants of wild type proteins.

For example, for 2C19 there are 11 currently known alleles - CYP2C19*1A, CYP2C19*1B, CYP2C19*2A, CYP2C19*2B, CYP2C19*3, CYP2C19*4, CYP2C19*5A, CYP2C19*5B, CYP2C19*6, CYP2C19*7, and CYP2C19*8. These and other CYP alleles are known in the art. One listing of the above and many other alleles is available on: http://www.imm.ki.se/CYPalleles/. There are currently over 20 known allelic variants for 3A4 and over 50 for 2D6.

A mutant is a P450 characterized by replacement or deletion of at least one amino acid from the wild type sequence. Such a mutant may be prepared for example by site-specific mutagenesis, or incorporation of natural or unnatural amino acids. Thus a mutant is a P450 polypeptide which is obtained by replacing at least one amino acid residue in a native or synthetic P450 with a different amino acid residue and/or by adding and/or deleting amino acid residues within the native polypeptide or at the N- and/or C-terminus of a polypeptide corresponding to P450.

To produce mutants, amino acids present in the said protein can be replaced by other amino acids having similar properties, for example hydrophobicity, hydrophobic moment, antigenicity, propensity to form or break α-helical or β-sheet structures, and so. Substitutional variants of a protein are those in which at least one amino acid in the protein sequence has been removed and a different residue inserted in its place. Amino acid substitutions are typically of single residues but may be clustered depending on functional constraints. Preferably amino acid substitutions will comprise conservative amino acid substitutions. Insertional amino acid variants are those in which one or more amino acids are introduced. This can be amino-terminal and/or carboxy-terminal fusion as well as intrasequence.

Amino acid substitutions, deletions and additions which do not significantly interfere with the three-dimensional structure of the P450 will depend, in part, on the region of the P450 where the substitution, addition or deletion occurs. In highly variable regions of the molecule, non-conservative substitutions as well as conservative substitutions may be tolerated without significantly disrupting the three-dimensional structure of the molecule. In highly conserved regions, or regions containing significant secondary structure, conservative amino acid substitutions are preferred.

Conservative amino acid substitutions are well-known in the art, and include substitutions made on the basis of similarity in polarity, charge, solubility, hydrophobicity, hydrophilicity and/or the amphipathic nature of the amino acid residues involved. For example, negatively charged amino acids include aspartic acid and glutamic acid; positively charged amino acids include lysine and arginine; amino acids with uncharged polar head groups having similar hydrophilicity values include the following: leucine, isoleucine, valine; glycine, alanine; asparagine, glutamine; serine, threonine; phenylalanine, tyrosine. Other conservative amino acid substitutions are well known in the art.

In some instances, it may be particularly advantageous or convenient to substitute, delete and/or add amino acid residues to a P450 binding pocket or catalytic residue in order to provide convenient cloning sites in cDNA encoding the polypeptide, to aid in purification of the polypeptide, etc. Such substitutions, deletions and/or additions which do not substantially alter the three dimensional structure of P450 will be apparent to those having skills in the art.

Mutants desirably exhibit the enzymatic activity of the P450 wild type protein from which they were derived.

In addition, the P450 may comprise a polypeptide tag allowing for affinity purification. A polyhistidine tag, having from 4 to 10 histidine residues is suitable for this purpose. Other tags include a glutathione S-transferase tag (GST), a streptavadin tag, a MBP (maltose binding protein) tag, a CBD (cellulose binding domain) tag, or an epitope tag such as an HA (hemagglutanin) tag or the like which can be bound by an antibody. Tags of this type are widely available in the art from academic and commercial sources.

The tag may be at the C- or N-terminus of the P450.

SEQ ID NO:2 shows the sequence of a truncated 2C19 P450 which we have used in the preparation of crystals. The invention in a further aspect provides an isolated protein which has the sequence of the truncated 2C19 shown herein, and crystals thereof.

### Host cell

The host cell in which the P450 is expressed is any suitable host cell which a person of skill in the art wishes to use as a matter of experimental convenience. Cytochrome P450 molecules have been widely expressed in *E.coli,* and there are numerous vector systems for this host cell which may be used.

Other host cells include yeast, e.g. *S.cerevisiae,* insect or mammalian, e.g. CHO, cells. Expression systems for these and many other host cell types are widely available in the art.

Host cells may be constructed so that the P450 is expressed constitutively, or is induced.

Once the cells have been cultured to express P450, they may be recovered by standard techniques available in the art. A convenient means is to recover the cells by low-speed centrifugation such that the cells are pelleted intact.

The process of the present invention is suitable for batch cell culture, and batches of cells from 100 ml to 10 litres can be conveniently handled by current laboratory equipment, though larger batches, for example from 10 to 100 litres, are not excluded.

### Salt buffer

This is buffer with a high ionic strength which is used to suspend the cells. It is a buffer comprising a salt which is readily soluble to provide a buffer having a conductivity of from 12 to 110 mS/cm. Such a buffer is desirably a salt having a concentration in the 200 - 1000 mM range. Preferably the salt is a potassium or sodium salt of an anion. Desirably the anion may be chloride or phosphate. Potassium phosphate (KPi) is particularly preferred.

A preferred salt concentration is selected to provide a conductivity of 25 to 35 mS/cm, for example about 30 mS/cm. A particularly preferred salt concentration is around 500 mM, e.g. 500 ± 50 mM.

The buffer will be maintained at a pH range of from 6.5 to 8.0, preferably from 7.0 to 7.6. The buffer may contain other reagents used conventionally in the art for protein purification, such as glycerol, β-mercaptoethanol, DNase, pH buffering agents, histidine, imidazole and protease inhibitors.

### Cell lysis

Cells may be lysed by physical means, such as sonication, continuous flow cell disruption, or in a French press, such that the cell walls are broken and the contents of the cells dispersed in the salt buffer. To achieve this in a French press or continuous flow cell disruptor, this may be operated at 10,000 to 20,000 psi.

Cell debris is removed (for example by low-speed centrifugation at about 10,000-25,000 g (e.g. about 22,000 g) or a short high speed centrifugation to 70,000 g or the like; i.e. such that any whole cells are pelleted but not the membrane fraction). The debris (e.g. pelleted cells) may be subject to a further round of lysis, and the debris-free lysate from this further round combined with the lysate obtained previously.

The lysate is then ready to use directly in the next stage of the process, without the need to isolate a membrane fraction by ultracentrifugation.

### Detergent

Once the lysate has been obtained, it is desirable that the detergent be added to the lysate as soon as possible, taking account of the constraints of the experimental set up. This will mean that the lysate is brought into contact with the detergent within 1 hour, preferably within 30 minutes or less of the preparation of the debris-free lysate.

The detergents which may be used are those conventionally used in the art of molecular and cell biology for the recovery and processing of biological materials. A large number of different types of detergents are available for this purpose. Many of these detergents are those of a molecular weight range of from about 350 to 1000, such as from 400 to 800. They include anionic surfactants such as cholic acid or salts thereof (e.g. the sodium salt) and deoxycholic acid or salts thereof (e.g. the sodium salt) as well as zwitterionic surfactants such as CHAPS (3-[(3-cholamidopropyl)dimethylammonio]-1-propane sulphonate).

A particularly preferred class of detergents are non-ionic detergents. There are a wide variety of non-ionic detergents available in the art. Non-ionic detergents include octyl-β-D-glucopyranoside; ethers, such as C2-10 alkylphenol ethers, of polylethylene glycol; and alkyl polyoxyethylene. Such compounds may be of a molecular weight range of 500 - 800 Da, and include Nonident™ P40, IGEPAL CA630, C₁₂E₉, and Triton™ X-100, and the like, which are commercially available.

The detergent is added to provide a concentration of from 0.015% to 1.2% v/v of detergent in the lysate. The amount of detergent added is preferably in the range of 0.1 to 0.5%, more preferably about 0.15 to 0.4%, most preferably about 0.2 to 0.4%, such as about 0.3%.

It is added in a volume so that desirably, the ionic strength of the lysate does not decrease by more than 10%.

### Recovery of P450

We have found that the above high-salt-detergent lysate prepared in accordance with the invention provides for the recovery of P450 protein in monodisperse form at a level much higher than experienced to date in the art. As mentioned above, the presence of the high salt buffer rules out an immediate ionic exchange chromatography step, but affinity purification may be performed as the next step.

Affinity purification may take the form of providing an affinity support matrix in which a ligand for the P450 is attached. The support may be a resin, a bead (e.g. glass or polymer such as polystyrene), a magnetic bead, or the like. Where the P450 contains a tag, the ligand will be cognate to the tag, e.g. Ni-NTA for a histidine tag, biotin for a streptavadin tag, etc. The ligand may also be an antibody, either to an epitope tag such as an HA tag, or to an epitope of the P450.

The lysate is brought into contact with the affinity support under conditions for the P450 to bind to the support. After binding, the support is then rinsed. The rinse buffer should be a high-salt-detergent buffer, which may be the same or different to the lysate buffer. Preferably it is the same. If different, it will still have concentrations of salt and detergent as specified above.

After rinsing, the P450 is removed from the support. This may be done by packing the support into a column, and eluting the P450 using a high-salt-detergent buffer (either the same or different, as in the preceding paragraph) which is modified to provide for removal of the P450 from its ligand. For example, for Ni-NTA, the buffer may contain histidine or imidazole at a sufficient excess concentration to displace the His tag of the P450. Suitable competitors may be used for other types of tags.

### Desalting

The recovered P450 is then desalted by a rapid desalting process. We have found that a size exclusion column may be used for this purpose, with a flow rate such that the P450 is separated from the high salt concentration within 10-30, preferably within 10 minutes. The P450 is then recovered by elution through the column with a low salt buffer.

While not wishing to be bound by any one particular theory, we believe that whereas gradual desalting by, for example, dialysis, leads to aggregation and denaturation of P450, the rapid desalting process reduces aggregation to a significant degree.

The low salt buffer is preferably a similar salt to the high salt buffer described above, e.g. a sodium or potassium salt such as a chloride or phosphate, with potassium phosphate again being preferred. By "low salt", it is meant less than 50 mM, preferably less than 20 mM, and preferably about 10 mM. At this stage, it is not necessary to maintain detergent in the buffer.

### Further purification

It is desirable that after the desalting step, the preparation is subject to further purification promptly, i.e. without storage or freezing of the sample. This can be achieved by applying the desalted eluate directly to a further purification column. If not, the eluate from the desalting process is collected and applied within 1 hour to the column.

A number of techniques are known as such in the art for the further purification or concentration of protein preparations, and examples of these are outlined in the accompanying examples. They include weak cation exchange columns, such as carboxymethyl-Sepharose™, BioRex™70, carboxymethyl-Biogel™, and the like, and strong cation exchange columns such as MonoS, which may be used to further remove detergent. For example, the desalted cytochrome P450 may be directly applied to a CM Sepharose™ column (e.g. a 5 ml HiTrap column, Pharmacia), previously equilibrated with 10 mM KPi, pH 7.4, 20% glycerol, 0.2 - 2.0 mM DTT, 1 mM EDTA ("buffer 1"). The following step elution protocol may then be run on the AKTA FPLC system; wash with 10 - 20 column volumes of buffer 1 and then 10 - 20 column volumes of 10mM KPi, pH 7.4, 20% glycerol, 0.2 - 2.0 mM DTT, 1mM EDTA, 75 mM KCl or NaCl in order to remove any trace of detergent. The P450 is then eluted with the above latter buffer with KCl or NaCl concentration increased to 500 mM.

Optionally this is followed by a size exclusion column, e.g Superose™, Superdex™, Sephacryl™, and the like. The protein recovered from either the cation exchange or size exclusion step may be concentrated to provide a solution suitable for crystallisation or other use. A concentration of from 20 to 120, e.g. 20 to 80 mg/ml may be achieved by the use of the present invention.

### Crystal formation

A number of methods are known as such in the art for obtaining protein crystals. Conveniently, the final protein is concentrated to 10-60, e.g. 20-40 mg/ml in 10-100 mM potassium phosphate with high salt (e.g. 500 mM NaCl or KCl) by using concentration devices which are commercially available.

Crystallisation of the protein is set up by the 0.5-2 µl hanging drop method and the protein is crystallised by vapour diffusion at 5-25 °C against a range of vapour diffusion buffer compositions.

Typically the vapour diffusion buffer comprises 0 - 27.5%, preferably 2.5-27.5% PEG 1K-20 K, preferably 1-8K or PEG 2000MME-5000MME, preferably PEG 2000 MME, or 0-10% Jeffamine M-600 and/or 5-20%, e.g. 10-20% propanol or 15-20% ethanol or about 15%-30%, e.g. about 15% 2-methyl-2,4-pentanediol (MPD), optionally with 0.01 M -1.6 M salt or salts and/or 0-0.15, e.g. 0-0.1, M of a solution buffer and/or 0-35%, such as 0-15%, glycerol and/or 0-35% PEG300-400; but preferably:
10-25% PEG 1K-8K or PEG 2000MME or 0-10% Jeffamine M-600 and/or 5-15%, e.g. 10-15%, propanol or ethanol, optionally with 0.1 M -0.2 M salt or salts and/or 0-0.15, e.g. 0-0.1 M solution buffer and/or PEG400, but more preferably:
15-20% PEG 3350 or PEG 4000 or PEG 2000MME or 0-10% Jeffamine M-600 or 5-15%, e.g. 10-15% propanol or ethanol, optionally with 0.1 M -0.2 M salt or salts and/or 0-0.15 M solution buffer.

The salt may be an alkali metal (particularly lithium, sodium and potassium), alkaline earth metal (e.g. magnesium or calcium), ammonium, ferric, ferrous or transition metal salt (e.g. zinc) of a halide (e.g. bromide, chloride or fluoride), acetate, formate, nitrate, sulfate, tartrate, citrate or phosphate. This includes sodium fluoride, potassium fluoride, ammonium fluoride, ammonium acetate, lithium acetate, magnesium acetate, sodium acetate, potassium acetate, calcium acetate, zinc acetate, ammonium chloride, lithium chloride, magnesium chloride, potassium chloride, sodium chloride, potassium bromide, magnesium formate, sodium formate, potassium formate, ammonium formate, ammonium nitrate, lithium nitrate, potassium nitrate, sodium nitrate, ammonium sulfate, potassium sulfate, lithium sulfate, sodium sulfate, di-sodium tartrate, potassium sodium tartrate, di-ammonium tartrate, potassium dihydrogen phosphate, tri-sodium citrate, tri-potassium citrate, zinc acetate, ferric chloride, calcium chloride, magnesium nitrate, magnesium sulfate, sodium dihydrogen phosphate, di-sodium hydrogen phosphate, dipotassium hydrogen phosphate, ammonium dihydrogen phosphate, di-ammonium hydrogen phosphate, tri-lithium citrate, nickel chloride, ammonium iodide, di-ammonium hydrogen citrate.

Solution buffers if present include, for example, Hepes, Tris, imidazole, cacodylate, tri-sodium citrate/citric acid, tri-sodium citrate/HCl, acetic acid/sodium acetate, phosphate-citrate, sodium potassium phosphate, 2-(N-morpholino)-ethane sulphonic acid/NaOH (MES), CHES or bis-trispropane.

The pH range is desirably maintained at pH 4.2-8.5, preferably 4.7-8.5.

Crystals may be prepared using a Hampton Research Screening kit, Poly-ethylene glycol (PEG)/ion screens, PEG grid, Ammonium sulphate grid, PEG/ammonium sulphate grid or the like.

Crystallisation may also be performed in the presence of an inhibitor of P450, e.g. fluoroxamine or 2-phenyl imidazole.

Additives can be added to a crystallisation condition identified to influence crystallisation. Additive Screens are to be used during the optimisation of preliminary crystallisation conditions where the presence of additives may assist in the crystallisation of the sample and the additives may improve the quality of the crystal e.g. Hampton additive Screens which use glycerol, polyols and other protein stabilizing agents in protein crystallisation (R. Sousa. Acta. Cryst. (1995) D51, 271-277) or divalent cations (Trakhanov, S. and Quiocho, F.A. Protein Science (1995) 4,9, 1914-1919).

The invention is illustrated by the following examples.

### Expression vector

The expression vector pCWOri+, provided by Prof.F. W. Dahlquist, University of Oregon, Eugene, Oregon, was used to express the truncated human cytochrome P450s in the E. coli strain XL1 Blue (Stratagene). Cytochrome P450 cDNAs, modified at their 5'terminus, were introduced at the NdeI/SalI cloning sites in the polylinker of the pCWOri+ vector. Residues 2-29 of the native N terminus of human cytochrome P450s 2C9 and 2C19 that constitute the trans-membrane domain were substituted by the motif AKKTSSKGR (SEQ ID NO:9, Figure 1).
An alanine was introduced as second codon in order to improve the protein expression. A four histidine tag was inserted at the 3' terminus to facilitate the purification of the proteins in high salt buffers. The coding sequence of the 2C19 protein is shown as SEQ ID NO:1.

### Bacteria expression

A single ampicillin resistant colony of XL1 blue cells was grown overnight at 37°C in Terrific Broth (TB) with shaking to near saturation and used to inoculate fresh TB media. Bacteria were grown to an OD600nm =0.4 in TB broth containing 100 µg of ampicillin per ml at 37°C at 200 rpm. The haem precursor delta aminolevulinic acid (80mg/ml) was added 15 min prior induction with 1 mM IPTG and then the temperature was shifted to 30°C. The bacterial culture was continued under gentle agitation (185 rpm) at 30°C for 48 to 72 hours.

### Protein purification - example 1(a)

The cells were pelleted at 10000 xg for 10 min and resuspended in 500 mM KPi, pH7.4, 20% glycerol, 10 mM mercaptoethanol, 1:1000 dilution of protease inhibitor cocktail (Calbiochem), 0.01 mg/ml (about 40 U/ml) DNase 1 and 5 mM MgSO₄ The final volume is at most 50ml per L culture.

The cells were lysed by passing twice through a Constant Systems Cell Homogeniser at 12000 psi. The cell debris was then removed by centrifugation at 70000 xg at 4°C for 30 min.

Detergent IGEPAL CA630 (Sigma) was added drop by drop to the lysate to a final concentration of 0.3% (v/v) and the lysate was incubated with previously washed NiNTA resin (Qiagen) overnight at 4°C, using agitation. The NiNTA resin was pelleted by centrifugation at 2000 xg for 2min at 4°C and washed with 20 resin volumes of 500 mM KPi, pH7.4, 20% glycerol, 10 mM mercaptoethanol, 50 mM glycine, 1:1000 dilution of protease inhibitor cocktail, 0.3%(v/v) IGEPAL CA630 and the resin pelleted by centrifugation at 2000 xg for 2min at 4°C. The resin was washed with 10 resin volumes of 500 mM KPi, pH7.4, 20% glycerol, 10 mM mercaptoethanol, 7.5 mM Histidine, 1:1000 dilution protease inhibitors, 0.3% IGEPAL CA630 and the resin recovered by centrifugation as described above. Finally, the resin was packed into a column at 4°C and the cytochrome P450 eluted with 500 mM KPi, pH7.4, 20 % glycerol, 10 mM mercaptoethanol, 100 mM Histidine, 1:1000 dilution of protease inhibitor cocktail, 0.3%(v/v) IGEPAL CA630.

The cytochrome P450 obtained from the NiNTA column was quickly desalted (<10min) into 10 mM KPi, pH 7.4, 20% glycerol, 0.2 mM DTT, 1mM EDTA using a HiPrep 26/10 desalting column (Pharmacia) on the AKTA FPLC system (Pharmacia), at a flow rate of 5ml/min and collecting 16 ml fractions. The desalted cytochrome P450 was directly applied to a CM Sepharose column (Pharmacia), previously equilibrated with 10 mM KPi, pH 7.4, 20% glycerol, 0.2 mM DTT, 1 mM EDTA. The following step elution protocol is then run on the AKTA FPLC system; wash with 20 column volumes of 10mM KPi, pH 7.4, 20% glycerol, 0.2mM DTT, 1mM EDTA, 75 mM KCl in order to remove any trace of detergent and eluted with the above buffer with KCl concentration increased to 500 mM. Tables 1 and 2 show the recovery of 2C9 and 2C19 respectively through the above process.

**Table 1 - Purification table for 2C9**

| **Purification Stage** | **nmoles 2C19** | **% recovery** |
|---|---|---|
| **Lysate** | 708 | 100 |
| **NiNTA Pool** | 534 | 75.4 |
| **CM Sepharose Flow Through** | 6.7 | 0.95 |
| **CM Sepharose Pool** | 566 | 80.0 |

**Table 2 - Purification table for 2C19**

| **Purification Stage** | **nmoles 2C19** | **% recovery** |
|---|---|---|
| **Lysate** | 847 | 100 |
| **NiNTA 7.5 mM Histidine wash** | 130 | 15.0 |
| **NiNTA Pool** | 842 | 99.0 |
| **CM Sepharose Flow Through** | 7.0 | 0.9 |
| **CM Sepharose Pool** | 653 | 77.0 |

The P450 fractions were concentrated using a microconcentrator (Centriprep or Centricon 30). P450 crystals were obtained from this preparation.

In a repeat of this protocol, the P450 sample obtained from the CM Sepharose column was applied on the top of a Superose 6 HR10/30 gel filtration column (Pharmacia) and eluted at 0.2ml/min with buffer containing 100 mM KPi, pH7.4, 300 mM KCl, 20% glycerol, 0.2 mM DTT. The protein was collected and concentrated up to 40 mg/ml using a centricon for crystallisation assays.

### Protein purification - example 1(b).

P450 2C19 was expressed in bacteria as described above, and the cells were pelleted at 10000 xg for 10 min and resuspended in 500 mM KPi, pH7.4, 20 % glycerol, 10 mM mercaptoethanol, 1:1000 dilution of protease inhibitor cocktail (Calbiochem), 10 mM imidazole, 0.01 mg/ml DNase 1 and 5 mM MgSO₄. The final volume is at most 50 ml per L culture.

The cells were lysed by passing twice through a Constant Systems Cell Homogeniser at 12000 psi. The cell debris was then removed by centrifugation at 70000 xg at 4°C for 30 min.

Detergent IGEPAL CA630 (Sigma) was added drop by drop to the lysate to a final concentration of 0.3% (v/v) and the lysate was incubated with previously washed NiNTA resin (Qiagen) overnight at 4 °C, using agitation. The NiNTA resin was pelleted by centrifugation at 2000 xg for 2 min at 4°C and washed with 20 resin volumes of 500 mM KPi, pH 7.4, 20% glycerol, 10 mM mercaptoethanol, 10 mM imidazole, 1:1000 dilution of protease inhibitor cocktail, 0.3%(v/v) IGEPAL CA630 and the resin pelleted by centrifugation at 2000 xg for 2 min at 4°C. The resin was washed with 10 resin volumes of 500 mM KPi, pH7.4, 20% glycerol, 10 mM mercaptoethanol, 20 mM imidazole, 1:1000 dilution protease inhibitors, 0.3% IGEPAL CA630 followed by washing with 5 resin volumes of 500 mM KPi, pH7.4, 20% glycerol, 10 mM mercaptoethanol, 50 mM imidazole, 1:1000 dilution protease inhibitors, 0.3% IGEPAL CA630. Between the washes the resin was recovered by centrifugation as described above. Finally, the resin was packed into a column at 4°C and the cytochrome P450 eluted with 500 mM KPi, pH 7.4, 20% glycerol, 10 mM mercaptoethanol, 300 mM imidazole, 1:1000 dilution of protease inhibitor cocktail, 0.3%(v/v) IGEPAL CA630.

The P450 fraction was then concentrated substantially as described above using a Vivaspin 30 microconcentrator (equivalent to the Centriprep or Centricon 30 microconcentrators). The recovered fraction was used to prepare protein crystals. In a repeat of this protocol, the protein fraction was instead applied to a Superose 6 HR10/30 gel filtration column and eluted as before prior to crystallisation.

### Quality assays

The quality of the final preparation from CM Sepharose or Superose 6 was evaluated by:
SDS polyacrylamide gel electrophoresis was performed using commercial gels (Nugen) followed by CBB staining according to the manufacturer's instructions. The purity as estimated by scanning a digital image of a gel was estimated at least 95%.

Gel filtration chromatography using a Superose 6 HR10-30 column (Pharmacia) was performed to assess the aggregation state. The fractionation range for this column is 5x10³ to 5x10⁶ Da and is thus well adapted to the resolution of large complexes The column was eluted at 0.2ml/min with buffer containing 100 mM KPi, pH7.4, 300 mM KCl, 20% glycerol, 0.2 mM DTT. 0.2ml protein samples at a concentration of approximately 40 mg/ml were used. Absorbance at 280 nm was monitored and the peak was collected and analyzed using dynamic light scattering.

### Light scattering.

Samples (0.15 ml) were analysed by DLS in fluorimeter quartz cells at 90° using laser radiation at 830.3 nm. Data was collected using a log correlator with variable expansion spanning a wide dynamic range. All measurements were performed at 20 °C with samples collected immediately from the gel filtration column. Runs were usually on average of 10 runs of 10 sec each. To obtain an estimation of the molecular weight, we used a frictional ratio of 1.26 and a partial specific volume of 0.726.

Samples prepared using our new method of purification possessed a good solubility and an absence of significant aggregation as shown by:
- the ratio of far channel extrapolation and measured average scattering was always between 0.999 and 1.003.
- the average count rate did not vary significantly, with approximately 1% standard deviation.
- analysis of the autocorrelation function using bi exponential fitting showed that 2C9 had an estimated Mr of approximately 180 kDa and that 2C19 had an estimated Mr of approximately 160 kDa i.e. both are oligomers composed of no more than four subunits.
- good stability of the samples (over 24h) at 20°C.

Samples prepared by published protocols showed signs of a severe aggregation:
- large fluctuations of the scattered light intensity, with a standard deviation of more than 10%.
- analysis of the autocorrelation function showed very slow exponential decay an indication of the presence of large aggregates (Mr > 10⁶ Da), composed of a large number of P450 subunits. These samples also show a high degree of polydispersity.
- samples also showed further aggregation as a function of time.

These signs of severe aggregation in samples prepared by published methods were still present after sample filtration through 20 nm diameter pores or centrifugation at 200,000g for 30 min.

### Example 1 (c)

### Bacteria expression

Cells expressing P450 2C19 were grown as in 1(a) above.

### Protein purification

The cells were pelleted at 10000 g for 10 min and resuspended in a buffer containing 500 mM KPi, pH 7.4, 20 % glycerol, 10 mM mercaptoethanol, 0.1% (v/v) of protease inhibitor cocktail (Calbiochem), 10 mM imidazole, 40U/ml DNase 1 and 5 mM MgSO₄.

The cells were lysed by passing twice through a Constant Systems Cell Homogeniser at 10000 psi. The cell debris was then removed by centrifugation at 22000 g at 4 °C for 30 min.

Detergent IGEPAL CA630 (Sigma) was added dropwise from a 10% stock solution to the lysate at a final concentration of 0.3% (v/v) and the lysate was incubated with previously washed NiNTA resin (Qiagen) overnight at 4 °C, using agitation. The protein bound-NiNTA resin was pelleted by centrifugation at 2000 g for 2 min at 4 °C. The resin was washed with 20 resin volumes of 500 mM KPi, pH 7.4, 20% glycerol, 10 mM mercaptoethanol, 10 mM imidazole, 1:1000 dilution of protease inhibitor cocktail, 0.3%(v/v) IGEPAL CA630 and the resin pelleted by centrifugation at 2000 xg for 2 min at 4 °C. The resin was then washed with 10 resin volumes of 500 mM KPi, pH 7.4, 20% glycerol, 10 mM mercaptoethanol, 20 mM imidazole, 0.1% (v/v) protease inhibitors, 0.3% IGEPAL CA630 and the resin recovered by centrifugation as described above.

The resin was packed into a column at 4 °C and the cytochrome P450 eluted with 500 mM KPi, pH 7.4, 20 % glycerol, 10 mM mercaptoethanol, 300 mM imidazole, 0.1% (v/v) of protease inhibitor cocktail, 0.3%(v/v) IGEPAL CA630.

The cytochrome P450 obtained from the NiNTA column was quickly desalted into 10 mM KPi, pH 7.4, 20% glycerol, 2.0 mM DTT, 1 mM EDTA using a HiPrep 26/10 desalting column (Pharmacia), at a flow rate of 5 ml/min.

The desalted cytochrome P450 was directly applied to a CM Sepharose column (Pharmacia), previously equilibrated with 10 mM KPi, pH 7.4, 20% glycerol, 2.0 mM DTT, 1 mM EDTA. The following step elution was applied: wash with 20 column volumes of 10 mM KPi, pH 7.4, 20% glycerol, 2.0 mM DTT, 1 mM EDTA, wash with the above buffer with 75 mM KCl in order to remove any trace of detergent, then eluted with the above buffer with KCl concentration increased to 500 mM.

The protein was concentrated up to 40 mg/ml using a microconcentrator for crystallization assays.

### Functionality assays

Activity assays on P450 2C9 and 2C19 were performed using a fluorescent method using LS spectrometer (Perkin Elmer Instruments).

Twenty p moles of P450 2C9 were reconstituted with 0.1 unit of purified human oxidoreductase in presence of 100 µM of substrate methoxy-4-(trifluoromethyl)-coumarin, a NADPH regenerating system that includes 1.3 mM NADP⁺, 3.3 mM Glucose-6-phosphate and 0.1 unit glucose-6-phosphate dehydrogenase in 300 µl final volume of 25 mM KPi, pH7.4, 3.3 mM MgCl₂. Incubations were performed at 37 °C for several minutes and 7-hydroxy -4-(trifluoromethyl)-coumarin was used as metabolite standard to determinate the metabolic rate. The excitation and emission wavelengths used were respectively 409 and 530nm. The activity of 2C9 was determined to be 0.94 ± 0.09 nmol/min/nmol P450.

Activity assays on P450 2C19 were performed using 20 pmoles of P450 2C9 and reconstituted with 0.1 unit of purified human oxidoreductase in presence of 10 µM of substrate - dibenzylfluorescein, a NADPH regenerating system that includes 1.3 mM NADP⁺, 3.3 mM Glucose-6-phosphate and 0.1 unit glucose-6-phosphate dehydrogenase in 300 µl final volume of 25 mM KPi, pH7.4, 3.3 mM MgCl₂ at 37 °C. Hydroxy-dibenzylfluoresceinwas used as metabolite standard. The excitation and emission wavelengths used were respectively 485 and 538nm. The activity of 2C19 was determined to be 1.27 ± 0.06 nmol/min/nmol P450.

### Crystallisation

Crystallisation of P450 2C19 was achieved at 20 - 40 mg/ml protein against 0.1 M Hepes, pH6.0, 1.6 M ammonium sulphate, 2.5% PEG 6000. Crystals grew over a two week period as dark brown needles from an initial precipitate.

Further crystallisation of P450 2C19 was achieved at protein concentrations of 10 to 60 mg/ml against the following range of conditions:
0.15 M-0.2 M Ammonium Fluoride, 15%-20% PEG 3350;
0.15 M-0.2 M Sodium Fluoride, 15%-20% PEG 3350;
0.15 M-0.2 M Potassium Fluoride, 15%-20% PEG 3350;
0.15 M-0.2 M Ammonium Chloride, 15-20% PEG3350;
0.15 M-0.2 M Lithium Chloride, 15%-20% PEG 3350;
0.15 M-0.2 M Magnesium Chloride, 15%-20% PEG 3350;
0.15 M-0.3 M Sodium Chloride, 15%-20% PEG 3350;
0.15 M-0.2 M Potassium Chloride, 15%-20% PEG 3350;
0.15 M-0.2 M Sodium Nitrate, 15-20% PEG 3350;
0.15 M-0.2 M Lithium Nitrate, 15%-20% PEG 3350;
0.15 M-0.2 M Potassium Nitrate, 15%-20% PEG 3350;
0.15 M-0.2 M Ammonium Nitrate, 15%-20% PEG 3350;
0.15 M-0.2 M Magnesium Formate, 15%-20% PEG 3350;
0.15 M-0.2 M Sodium Formate, 15%-20% PEG 3350;
0.15 M-0.2 M Potassium Formate, 15%-20% PEG 3350;
0.15 M-0.2 M Ammonium Formate, 15%-20% PEG 3350;
0.15 M-0.2 M Ammonium Acetate, 15%-20% PEG 3350;
0.15 M-0.2 M Lithium Acetate, 15%-20% PEG 3350;
0.15 M-0.2 M Sodium Acetate, 15%-20% PEG 3350;
0.15 M-0.2 M Potassium Acetate, 15%-20% PEG 3350;
0.15 M-0.2 M Lithium Sulfate Monohydrate, 15%-20% PEG 3350;
0.15 M-0.2 M Sodium Sulfate Decahydrate, 15%-20% PEG 3350;
0.15 M-0.2 M Potassium Sulfate, 15%-20% PEG 3350;
0.15 M-0.2 M Ammonium Sulfate, 15%-20% PEG 3350;
0.15 M-0.2 M di-Sodium Tartrate, 15%-20% PEG 3350;
0.15 M-0.2 M Potassium Sodium Tartrate, 15%-20% PEG 3350;
0.15 M-0.2 M di-Ammonium Tartrate, 15%-20% PEG 3350;
0.15 M-0.2 M Potassium dihydrogen Phosphate, 15%-20% PEG 3350;
0.15 M-0.2 M tri-Potassium Citrate, 15%-20% PEG 3350; 20% PEG 3350;
0.05 - 0.2 M Imidazole pH 7.0-8.0, 10-20% 2-propanol, 0-15% PEG400;
0.1 M Tris pH 8.5, 20% PEG 1000;
0.1 M tri-Sodium Citrate pH 5.5-5.6, 20% PEG 3000;
0.1 M Tris pH 7.0-7.6, 15-25% PEG 2000MME;
0.1 M Hepes pH 7.2-7.6, 0-0.2 M NaCl, 20-25% PEG 3000;
0.1 M Imidazole pH 8.0, 0.2 M Calcium Acetate, 10% PEG 8000;
0.01 M Ferric Chloride hexahydrate, 0.1 M tri-Sodium Citrate dihydrate pH 5.6, 0-10% Jeffamine M-600;
0.1 M Sodium Cacodylate, pH 6.5, 0.2 M Ammonium Sulfate, 15-30% PEG 8000;
0.1 M Sodium Cacodylate, pH 6.5, 0.2 M Magnesium Acetate, 10-20% PEG 8000; and
0.1 M Sodium Cacodylate, pH 6.5, 0.2 M Zinc Acetate, 9-18% PEG 8000;
10% 2-Propanol;
0.1 M Tris pH 7.0-7.4, 15 % 2-propanol;
0.3 M Sodium Acetate, 0.1 M Imidazole-HCl pH 7.0, 25% MPEG 2K;
0.2 M Lithium Sulphate, 0.1 M Imidazole-HCl pH 7.0, 25% MPEG 2K;
0.2 M Potassium Bromide, 0.1 M Imidazole-HCl pH 7.0, 25% MPEG 2K;
0.1 M HEPES, pH 7.0-7.5; 5-10% 2-propanol, 10-20% PEG 4000, 0-15% glycerol;
0.1 M Tris pH 7.0-8.2, 15-20 % ethanol;
0.1 M Tris pH 7.0-7.6, 20-27.5 % PEG 3000;
0.1 M HEPES, pH 7.2-7.4; 15-20 % PEG 8000;
0.1 M Cacodylate, pH 7.0; 15-25 % PEG 2000MME;
0.1 M Imidazole, pH 7.0, 15-25 % PEG 2000MME;
0.1 M Tris pH 7.0-7.6, 0-0.2 M Calcium acetate, 20-25 % PEG 3000;
0.1 M HEPES pH 7.4, 0-0.1 M Calcium acetate, 20 % PEG 3000; 0.1 M HEPES pH 7.0-7.2, 20% PEG 2000MME;
0.05-0.085 M Tris pH 8.4-8.5, 0.2 M Lithium sulphate, 25-25.5% PEG 4K, 0-15 Glycerol;
0.05-0.25 M K2 H PO4, 15-25% PEG 3350, 0-10% glycerol ; 0.10 M Sodium Cacodylate, pH 6.6, 20% PEG 3350;
0.10 M Sodium Cacodylate, pH 6.5, 0.20 M Magnesium Acetate, 15% MPD;
0.05 M KH2PO4, 10% PEG 8000;
0.10 M acetate, pH 4.5, 0.20 M Zinc acetate, 20% PEG 1000;
0.10 M acetate, pH 4.5, 0.20 M calcium acetate, 30% PEG 400;
0.10 M acetate, pH 4.5, 0.20 M sodium chloride, 1.26 M Ammonium Sulphate;
0.10 M Tris-HCl, pH 8.5, 0.20 M Magnesium Chloride, 15% PEG 4000;
0.10 M HEPES, pH 7.5, 0.20 M Magnesium Chloride, 15% Iso-Propanol;
0.10 M HEPES, pH 7.5, 0.20 M Magnesium Chloride, 15% PEG 400;
0.10 M HEPES, pH 7.5, 0.75 M Lithium sulphate;
0.10 M Phosphate-citrate, pH 4.2, 0.20 M Lithium sulphate, 20% PEG 1000;
0.10 M Phosphate-citrate, pH 4.2, 0.20 M Sodium chloride, 10% PEG 3000;
0.10 M Phosphate-Citrate pH 4.2, 0.20 M Ammonium Sulphate, 20% PEG 300, 10% Glycerol;
0.085 M Tri-Sodium Citrate Dihydrate pH 5.6, 0.2 M Ammonium Acetate, 25.5% PEG 4K, 15% Glycerol;
0.10 M Tri-Sodium Citrate Dihydrate, pH 5.6, 0.20 M Ammonium Acetate, 15% PEG 4000;
0.1 M Tri-Sodium Citrate-HCl, pH 5.6, 10% PEG 4000, 10% Isopropanol;
0.10 M sodium citrate, pH 5.5-5.6, 20% PEG 3000;
0.10 M Na/K phosphate, pH 6.2, 0.20 M sodium chloride, 20% PEG 1000;
0.10 M MES, pH 6.0, 0.20 M Zinc acetate, 10% PEG 8000.

Crystals grew over a two week period in a range of morphologies - dark brown needles, hexagonal rods or ellipsoids.

Analylsis of the crystals by x-ray crystallography revealed that the crystals of the 2C19 truncated protein had cell dimensions of a=158Å, b=158Å, c=212Å, α=90°, β=90°, γ=120°, and a space group P321. Cell dimensions may vary by 1 to 2 Å upon repeat crystallisation, and reference to these dimensions includes reference to such variations.

Crystallisation of 2C9 was obtained at 20 mg/ml in 200 mM ammonium sulphate, 100 mM MES, pH 6.0, 5-30% PEG 6000. Small crystals of 2C9 appeared over two weeks as chunky brown blocks.

### Cocrystallisation of 2C19

2C19 was cocrystallised with an inhibitor under a range of conditions. A ten fold molar excess (typically 3.7 - 7.4 mM) of inhibitor e.g. fluvoxamine or 2-phenylimidazole suspended in water or ethanol was added to a solution of typically 20 - 40 mg/ml 2C19 P450 protein. The resulting mixture was incubated at 4°C for 10-60 minutes. The resulting inhibitor-protein mixture was used in crystallisation trials using protocols selected from those described above.

### Example 2: Crystallisation of 2C19-1B

The wild type 2C19*1B cDNA previously characterized by Richardson et al. (Arch. Biochem. Biophys. 323(1):87-96 (1995)) was produced. Originally the double mutant 2C19*1B R150H D414H (clone 2C19 above) had been isolated and expressed. This mutant was then reverted in two steps to the wild type 2C19*1B sequence (shown as SEQ ID NO:3 and SEQ ID NO:4 below) by site directed mutagenesis, using the Quick Change kit from Stratagene.

### Construction of 2C19-1B

The expression vector pCWOri+, provided by Prof. F. W. Dahlquist, University of Oregon, Eugene, Oregon, USA, was used to express the truncated human cytochrome P450 in the *E*. *coli* strain XL1 Blue (Stratagene).

The wild type clone 2C19-1B, based on the wild type 2C19*1B cDNA previously characterized by Richardson et al. (Arch. Biochem. Biophys. 323(1):87-96 (1995)), was produced by correcting the two mutations that are present in the clone 2C19 in two steps by site directed mutagenesis, using the Quick Change kit from Stratagene. The reversion H150R was performed using the following sets of complementary oligonucleotides 5'CAAGAGGAAGCCCGCTGCCTTGTGGAGGAG3'(SEQ ID NO:12) and 5'CTCCTCCACAAGGCAGCGGGCTTCCTCTTG3' (SEQ ID NO:13).

The reversion H414D was performed using the following set of complementary oligonucleotides 5′CCCTCGTCACTTTCTGGATGAAGGTGGAAATTTTAAG3′ (SEQ ID NO:14) and

5′CTTAAAATTTCCACCTTCATCCAGAAAGTGACGAGGG3′ (SEQ ID NO:15).

The reverted codons are underlined.

### Bacteria expression

A single ampicillin resistant colony of XL1 blue cells was grown overnight at 37 °C in Terrific Broth (TB) with shaking to near saturation and used to inoculate fresh TB media. Bacteria were grown to an OD600nm =0.4 in 1 liter of TB broth containing 100 µg/ml of ampicillin at 37°C at 185 rpm in 2 liter flask. The heme precursor delta aminolevulinic acid (80 mg/l) was added 30 min prior to induction with 1 mM isopropyl-β-D-thiogalactopyranoside (IPTG) and the temperature lowered to 25 °C. The bacterial culture was continued under agitation at 25 °C for 72 hours.

### Protein purification

The cells were pelleted at 10000 g for 10 min and resuspended in a buffer containing 500 mM KPi, pH 7.4, 20 % glycerol, 10 mM mercaptoethanol, 0.1% (v/v) of protease inhibitor cocktail (Calbiochem), 10 mM imidazole, 40U/ml DNase 1 and 5 mM MgSO₄.

The cells were lysed by passing twice through a Constant Systems Cell Homogeniser at 10000 psi. The cell debris was then removed by centrifugation at 22000 x g at 4 °C for 30 min.

Detergent IGEPAL CA630 (Sigma) was added dropwise from a 10% stock solution to the lysate at a final concentration of 0.3% (v/v) and the lysate was incubated with previously washed NiNTA resin (Qiagen) overnight at 4 °C, using agitation. The protein bound-NiNTA resin was pelleted by centrifugation at 2000 g for 2 min at 4 °C. The resin was washed with 20 resin volumes of 500 mM KPi, pH 7.4, 20% glycerol, 10 mM mercaptoethanol, 10 mM imidazole, 1:1000 dilution of protease inhibitor cocktail, 0.3%(v/v) IGEPAL CA630 and the resin pelleted by centrifugation at 2000 xg for 2 min at 4 °C. The resin was then washed with 10 resin volumes of 500 mM KPi, pH 7.4, 20% glycerol, 10 mM mercaptoethanol, 20 mM imidazole, 0.1% (v/v) protease inhibitors, 0.3% IGEPAL CA630 and the resin recovered by centrifugation as described above.

The resin was packed into a column at 4 °C and the cytochrome P450 eluted with 500 mM KPi, pH 7.4, 20 % glycerol, 10 mM mercaptoethanol, 300 mM imidazole, 0.1% (v/v) of protease inhibitor cocktail, 0.3%(v/v) IGEPAL CA630.

The cytochrome P450 obtained from the NiNTA column was quickly desalted into 10 mM KPi, pH 7.4, 20% glycerol, 2.0 mM DTT, 1 mM EDTA using a HiPrep 26/10 desalting column (Pharmacia), at a flow rate of 5 ml/min.

The desalted cytochrome P450 was directly applied to a CM Sepharose column (Pharmacia), previously equilibrated with 10 mM KPi, pH 7.4, 20% glycerol, 2.0 mM DTT, 1 mM EDTA. The following step elution was applied: wash with 20 column volumes of 10 mM KPi, pH 7.4, 20% glycerol, 2.0 mM DTT, 1 mM EDTA, wash with the above buffer with 75 mM KCl in order to remove any trace of detergent, then eluted with the above buffer with KCl concentration increased to 500 mM.

The protein was concentrated up to 40 mg/ml using a microconcentrator for crystallization assays.

### Crystallization of 2C19-1B

Crystals of the 2C19-1B were grown using the hanging drop vapor diffusion method. Protein at 40 mg/ml in 10mM Kpi pH 7.4, 0.5 M KCl, 2mM DTT, 1mM EDTA. 20% glycerol, was mixed in a 1:1 ratio, using 0.5ul drops, with a reservoir solution. The crystals of 2C19-1B grew over a reservoir solution containing:
0 .1 M K2HPO4, 25% PEG 3350
0.15 M K2HPO4, 20% PEG 3350
0 .15 M K2HPO4, 25% PEG 3350
0.2 M K2HPO4, 20% PEG 3350
0.2 M K2HPO4, 25% PEG 3350
0.25 M K2HPO4, 20% PEG 3350
0.25 M K2HPO4, 25% PEG 3350
0.1 M Tris-HCl pH 8.4, 0.1 M Lithium Sulphate, 15% PEG 4000
0.05 M Tris-HCl pH 8.4, 0.2 M Lithium Sulphate, 20% PEG 4000
0.05 M Tris-HCl pH 8.4, 0.2 M Lithium Sulphate, 25% PEG 4000
0.1 M Tris-HCl pH 7, 20% MPEG 2000
0.1 M HEPES pH 7.5, 0.2 M sodium chloride 20% PEG 3000
0.1 M Imidazole-HCl pH 8, 10% Iso-Propanol
0.1 M Tris-HCl pH 7, 20% Ethanol

Crystals formed within 1 day at 25 °C, and had morphologies of needles, rods and hexagonal crystals.

### Example 3 - Purification and Crystallisation of 2D6

### 2D6 Construct Design

The heterologous expression of human cytochrome P450s often produces low yields of appropriately folded protein. In addition cytochrome P450s contain a membrane spanning domain and many flexible loops. These factors can hamper the production of sufficient protein for structural determination, or the ability of the protein to crystallize. One reason for low expression may be the differential coding bias of human genes as appose to the *E.coli* hosts used for expression. In order to determine if this had any effect on expression a codon optimized version of a truncated his tagged form of human cytochrome P450 2D6 was produced. This construct was also designed to remove the membrane spanning domain of the protein, introduce a C terminal his tag and, at a DNA level, incorporate appropriately spaced restriction sites for use in the production of mutated forms of the protein.

The protein coding sequence of the human cytochrome P450 2D6 was obtained from a public access database (SwissProt P10635).

The membrane spanning domain of this protein was then removed (residues 1-33), a leader sequence added to aid expression (makktsskgr) and a glycine, an alanine and a four histidine tag was added to the C terminus of the molecule. The glycine and alanine were added to incorporate a sacI restriction site to be used for altering the tag. This resulted in the desired protein sequence (SEQ ID NO:6).

This protein sequence was then reverse transcribed using EditSeq, part of the Lasergene suite of programs. The program contains an option to reverse transcribe with the most frequently used codons in *E.coli.* This yielded a reverse transcribed DNA sequence. A degenerate form of the sequence was produced (one in which all the possible codon usages is represented) and this sequence was scanned for the presence of restriction sites not present in the pCW vector used for expression (i.e. sites unique to the insert). The codon usage of the gene was then altered to ensure that unique restriction sites were added at approx. 100bp gaps throughout the coding sequence (without changing the protein coded for). In this way it was hoped that the distribution of unique restriction sites would allow the easy removal/substitution of any part of the protein. This resulted in the desired gene sequence (SEQ ID NO:5) which was generated by gene assembly.

The DNA sequence and protein sequence of the 2D6 used in the invention are shown SEQ ID NO:5 and 6. In a further aspect, the invention provides a nucleic acid, preferably a DNA, having the sequence of SEQ ID NO:5. The DNA may be contained in an expression vector for expression of 2D6. The expression vector is preferably a bacterial expression vector designed for expression of 2D6 in a bacterial, particularly *E*. *coli,* host cell.

The resulting host cell transformed with this DNA encoding the optimised 2D6 in pCW vector showed surprisingly high levels of expression (50-90 mg/L), which is particularly advantageous for structural studies.

As with the proteins in Example 1 above, the N-truncation of the P450 results in an N-terminal sequence of MAKKTSSKGR (SEQ ID NO:10).

### Experimental

### Construct Generation

The protein coding sequence of the human cytochrome P450 2D6 was obtained from a public access database (SwissProt P10635).

The membrane spanning domain of this protein was then removed (residues 1-33), a leader sequence added to aid expression (makktsskgr, SEQ ID NO:10) and a glycine, an alanine and a four histidine tag was added to the C terminus of the molecule (note: the glycine and alanine were added to incorporate a sacI restriction site to be used for altering the tag). This resulted in the desired protein sequence (SEQ ID NO:6).

This protein sequence was then reverse transcribed using EditSeq, part of the Lasergene suite of programs. The program contains an option to reverse transcribe with the most frequently used codons in *E.coli.* This yielded a reverse transcribed DNA sequence. A degenerate form of the sequence was produced (one in which all the possible codon usages is represented) and this sequence was scanned for the presence of restriction sites not present in the pCW vector used for expression (i.e. sites unique to the insert). The codon usage of the gene was then altered to ensure that unique restriction sites were added at approx. 100bp gaps throughout the coding sequence (without changing the protein coded for). In this way it was hoped that the distribution of unique restriction sites would allow the easy removal/substitution of any part of the protein. This resulted in the desired gene sequence (SEQ ID NO:5).

The sequence for the codon optimised 2D6 sequence described above was split into 50bp oligos on both DNA strands. The oligos were designed so that they would overlap by 25bp with the oligos from the opposite strand.

Oligos used for the 2D6 assembly (all oligos, SEQ ID NOs:16-71) are shown 5'-3')

2D6ass5'1  CATATGGCTAAAAAAACCTCTTCTAAAGGCCGACCGCCGGGTCCGCTGCC
2D6ass5'2  GCTGCCAGGCCTGGGTAACCTGCTGCATGTGGACTTCCAGAACACCCCGT
2D6ass5'3  ACTGCTTCGACCAGCTGCGTCGTCGTTTCGGTGACGTGTTCTCTCTGCAG
2D6ass5'4  CTGGCTTGGACCCCGGTTGTTGTTCTGAACGGTCTGGCTGCTGTTCGCGA
2D6ass5'5  AGCTCTGGTTACCCACGGTGAAGACACCGCTGACCGTCCGCCGGTCCCGA
2D6ass5'6  TCACCCAGATCCTGGGTTTTGGTCCGCGTTCCCAAGGTGTTTTCCTGGCT
2D6ass5'7  CGTTACGGACCGGCTTGGCGTGAACAGCGTCGTTTCTCTGTTTCTACCCT
2D6ass5'8  GCGTAACCTGGGTCTGGGTAAAAAATCTCTGGAACAGTGGGTTACCGAAG
2D6ass5'9  AAGCTGCATGCCTGTGCGCTGCTTTCGCTAACCACTCTGGTCGTCCGTTC
2D6ass5'10 CGTCCGAACGGTCTGCTGGACAAAGCTGTTTCTAACGTTATCGCTTCTCT
2D6ass5'11 GACCTGCGGCCGCCGTTTCGAATACGACGACCCGCGTTTCCTGCGTCTGC
2D6ass5'12 TGGACCTGGCTCAGGAAGGTCTGAAAGAGGAGTCTGGTTTCCTGCGTGAA
2D6ass5'13 GTTCTGAACGCTGTTCCGGTTCTGCTGCACATCCCAGCTCTGGCTGGTAA
2D6ass5'14 AGTTCTGCGTTTCCAGAAAGCATTCCTGACCCAGCTGGACGAACTGCTGA
2D6ass5'15 CCGAACACCGTATGACCTGGGACCCGGCTCAGCCGCCACGTGACCTGACC
2D6ass5'16 GAAGCTTTCCTGGCTGAAATGGAAAAAGCTAAAGGTAACCCGGAATCTTC
2D6ass5'17 TTTCAACGATGAAAATCTGCGTATCGTTGTTGCTGACCTGTTCTCCGCGG
2D6ass5'18 GTATGGTTACCACCTCTACCACCCTGGCTTGGGGTCTGCTGCTGATGATC
2D6ass5'19 CTGCACCCGGATGTACAGCGTCGTGTTCAGCAGGAAATCGACGACGTTAT
2D6ass5'20 TGGCCAGGTTCGTCGGCCGGAAATGGGTGACCAGGCTCACATGCCGTACA
2D6ass5'21 CCACCGCTGTTATCCACGAAGTTCAGCGCTTCGGTGACATCGTTCCGCTG
2D6ass5'22 GGTATGACCCACATGACCTCTCGTGACATCGAAGTTCAGGGTTTCCGTAT
2D6ass5'23 CCCGAAAGGTACCACCCTGATCACCAACCTGTCTTCTGTTCTGAAAGACG
2D6ass5'24 AAGCTGTTTGGGAAAAACCGTTCCGTTTCCATCCGGAACACTTCCTGGAC
2D6ass5'25 GCTCAGGGTCACTTCGTTAAACCGGAAGCTTTCCTGCCGTTCTCTGCTGG
2D6ass5'26 TCGTCGTGCTTGCCTGGGTGAACCGCTGGCTCGTATGGAACTGTTCCTGT
2D6ass5'27 TCTTCACCTCTCTGCTGCAGCACTTCTCTTTCTCTGTTCCGACCGGTCAG
2D6ass5'28 CCGCGTCCGTCTCACCACGGTGTTTTCGCTTTCCTGGTTTCTCCGTCTCC
2D6ass3'1
GTCGACTCAGTGGTGGTGGTGAGCTCCACGCGGAACAGCGCACAGTTCGTACGGAGACGGAG
AAACCAGGAAAGCGA
2D6ass3'2  AAACACCGTGGTGAGACGGACGCGGCTGACCGGTCGGAACAGAGAAAGAG
2D6ass3'3  AAGTGCTGCAGCAGAGAGGTGAAGAACAGGAACAGTTCCATACGAGCCAG
2D6ass3'4  CGGTTCACCCAGGCAAGCACGACGACCAGCAGAGAACGGCAGGAAAGCTT
2D6ass3'5  CCGGTTTAACGAAGTGACCCTGAGCGTCCAGGAAGTGTTCCGGATGGAAA
2D6ass3'6  CGGAACGGTTTTTCCCAAACAGCTTCGTCTTTCAGAACAGAAGACAGGTT

2D6ass3'7 GGTGATCAGGGTGGTACCTTTCGGGATACGGAAACCCTGAACTTCGATGT
2D6ass3'8 CACGAGAGGTCATGTGGGTCATACCCAGCGGAACGATGTCACCGAAGCGC
2D6ass3'9 TGAACTTCGTGGATAACAGCGGTGGTGTACGGCATGTGAGCCTGGTCACC
2D6ass3'10 CATTTCCGGCCGACGAACCTGGCCAATAACGTCGTCGATTTCCTGCTGAA
2D6ass3'11 CACGACGCTGTACATCCGGGTGCAGGATCATCAGCAGCAGACCCCAAGCC
2D6ass3'12 AGGGTGGTAGAGGTGGTAACCATACCCGCGGAGAACAGGTCAGCAACAAC
2D6ass3'13 GATACGCAGATTTTCATCGTTGAAAGAAGATTCCGGGTTACCTTTAGCTT
2D6ass3'14 TTTCCATTTCAGCCAGGAAAGCTTCGGTCAGGTCACGTGGCGGCTGAGCC
2D6ass3'15 GGGTCCCAGGTCATACGGTGTTCGGTCAGCAGTTCGTCCAGCTGGGTCAG
2D6ass3'16 GAATGCTTTCTGGAAACGCAGAACTTTACCAGCCAGAGCTGGGATGTGCA
2D6ass3'17 GCAGAACCGGAACAGCGTTCAGAACTTCACGCAGGAAACCAGACTCCTCT
2D6ass3'18 TTCAGACCTTCCTGAGCCAGGTCCAGCAGACGCAGGAAACGCGGGTCGTC
2D6ass3'19 GTATTCGAAACGGCGGCCGCAGGTCAGAGAAGCGATAACGTTAGAAACAG
2D6ass3'20 CTTTGTCCAGCAGACCGTTCGGACGGAACGGACGACCAGAGTGGTTAGCG
2D6ass3'21 AAAGCAGCGCACAGGCATGCAGCTTCTTCGGTAACCCACTGTTCCAGAGA
2D6ass3'22 TTTTTACCCAGACCCAGGTTACGCAGGGTAGAAACAGAGAAACGACGCT
2D6ass3'23 GTTCACGCCAAGCCGGTCCGTAACGAGCCAGGAAAACACCTTGGGAACGC
2D6ass3'24 GGACCAAAACCCAGGATCTGGGTGATCGGACCGGCGGACGGTCAGCGGT
2D6ass3'25 GTCTTCACCGTGGGTAACCAGAGCTTCGCGAACAGCAGCCAGACCGTTCA
2D6ass3'26 GAACAACAACCGGGGTCCAAGCCAGCTGCAGAGAGAACACGTCACCGAAA
2D6ass3'27 CGACGACGCAGCTGGTCGAAGCAGTACGGGGTGTTCTGGAAGTCCACATG
2D6ass3'28 CAGCAGGTTACCCAGGCCTGGCAGCGGCAGCGGACCCGGCGGTCGGCCTT

The gene was assembled in four parts in an attempt to minimise errors introduced by PCR. The oligos used were 2D6ass5' 1-7 and 2D6ass3'22-28, 2D6ass5'6-15 with 2D6ass3'12-22, 2D6ass5'15-21 with 2D6ass3'8-14 and 2d6ass5'20-28 with 2D6ass3'1-9.

The oligos were resuspended to 100pM/microliter in sterile water and 5 microliters of each oligo was added to an eppendorf. This mix was then subjected to the following PCR cycling in varying quantities (1,2,4 microliters)
Step 1 40°C for 2 minutes
Step 2 72°C for 10 seconds
Step 3 94°C for 15 seconds
Step 4 40°C for 30 seconds
Step 5 72°C for 20 seconds plus 2 seconds per cycle
Step 6 4°C hold
with Steps 3-5 repeated 39 times.

The four gene fragments were then recovered by using the terminal primers of each assembly in a recovery PCR to enrich for the full length product. These reactions followed the cycling parameters
Step 1 95°C for 2 minutes
Step 2 95°C for 30 seconds
Step 3 57/60°C for 30 seconds
Step 4 72°C for 1 minute
Step 5 72°C for 10 minutes
Step 6 4°C hold
with Steps 2-4 repeated 25 times.

These gene fragments were then incubated with Taq polymerase at 72°C for 10 minutes in order to add A overhangs at the ends of the DNA. This process allowed the TOPO cloning of the fragments into the vector pCR4 (Invitrogen).

The TOPO cloned fragments were then subjected to DNA sequencing in order to verify they were correct. Of the four gene build fragments 3 had the correct sequence and one contained a frameshift mutation. The pCR4 clones containing gene portions 2D6ass5'15-21 and 2D6ass5'20-28 were digested with restriction enzymes AfeI and NcoI (NEB). The 2D6ass5'15-21 AfeI NcoI fragment was then ligated into 2D6ass5'20-28 vector to produce a pCR4 clone containing the portion of the gene spanning from oligos 2D6ass5'15-28.

The gene portion containing 5'6-15 was digested using the restriction enzymes BlpI and PciI and inserted into the pCR4 clone 2D6ass5'15-28 which had been digested with the same restriction endonucleases. This created a gene fragment spanning oligos 2D6ass5'6-28.

The pCR4 vector containing the gene portion spanning oligos 2D6ass5'1-7 was digested with restriction endonucleases NdeI and RsrII and the pCR4 clone containing the portion of the gene spanning from oligos 2D6ass5'15-28 was digested with restriction endonucleases RsrII and SalI. The two DNA fragments containing the 2D6 sequence were then three part ligated into a pCWori+ vector digested with the restriction endonucleases NdeI and SalI. All ligations were done as per the instructions in the Rapid Ligation Kit (Roche).
The resultant 2D6 codon optimised sequence contained one frameshift error and this was subsequently corrected using the Quickchage mutagenesis protocol (Stratagene) and the primers shown below.

(All oligos are shown 5'-3')
2D6 RT+RS 5'   GTAACCTGGGTCTGGGTAAAAATCTCTG (SEQ ID NO:72)
2D6 RT+RS 3'   CAGAGATTTTTACCCAGACCCAGGTTAC (SEQ ID NO:73)

An aliquot of the corrected product was used to transform *E*. *coli* XL1 Blue strain to yield the plasmids pCW-2D6 that encodes for the amino-terminal truncated 2D6.

### 2D6 Bacteria expression

A single ampicillin resistant colony of XL1 blue cells was grown overnight at 37 °C in Terrific Broth (TB) with shaking to near saturation and used to inoculate fresh TB media. Bacteria were grown to an OD600nm =0.5 in 1 litre of TB broth containing 100 µg/ml of ampicillin at 37°C at 185 rpm in 2 litre flask. The heme precursor delta aminolevulinic acid (80 mg/l) was added 30 min prior to induction with 1 mM isopropyl-P-D-thiogalactopyranoside (IPTG) and the temperature lowered to 25 °C. The bacterial culture was continued under agitation at 25 °C for 48 to 72 hours.

### 2D6 Protein purification

The cells were pelleted at 10000 g for 10 min and resuspended in a buffer containing 500 mM KPi, pH 7.4, 20 % glycerol, 10 mM mercaptoethanol, 0.1% (v/v) of protease inhibitor cocktail (Calbiochem), 10 mM imidazole, 40U/ml DNase 1 and 5 mM MgSO₄.

The cells were lysed by passing twice through a Constant Systems Cell Homogeniser at 10000 psi. The cell debris was then removed by centrifugation at 22000 g at 4 °C for 30 min.

Detergent IGEPAL CA630 (Sigma) was added dropwise from a 10% stock solution to the lysate at a final concentration of 0.15% (v/v) and the lysate was incubated with previously washed NiNTA resin (Qiagen) overnight at 4 °C, using agitation. The protein bound-NiNTA resin was pelleted by centrifugation at 2000 g for 2 min at 4 °C. The resin was washed with 20 resin volumes of 500 mM KPi, pH 7.4, 20% glycerol, 10 mM mercaptoethanol, 10 mM imidazole, 1:1000 dilution of protease inhibitor cocktail, 0.15%(v/v) IGEPAL CA630 and the resin pelleted by centrifugation at 2000 xg for 2 min at 4 °C. The resin was then washed with 10 resin volumes of 500 mM KPi, pH 7.4, 20% glycerol, 10 mM mercaptoethanol, 20 mM imidazole, 0.1% (v/v) protease inhibitors, 0.15% IGEPAL CA630 and the resin recovered by centrifugation as described above.

The resin was packed into a column at 4 °C and the cytochrome P450 eluted with 500 mM KPi, pH 7.4, 20 % glycerol, 10 mM mercaptoethanol, 300 mM imidazole, 0.1% (v/v) of protease inhibitor cocktail, 0.15%(v/v) IGEPAL CA630.

The cytochrome P450 obtained from the NiNTA column was quickly desalted into 10 mM KPi, pH 8.0, 20% glycerol, 2.0 mM DTT, 1 mM EDTA using a HiPrep 26/10 desalting column (Pharmacia), at a flow rate of 5 ml/min.

The desalted cytochrome P450 was directly applied to a CM Sepharose column (Pharmacia), previously equilibrated with 10 mM KPi, pH 8.0, 20% glycerol, 2.0 mM DTT, 1 mM EDTA. The following step elution was applied: wash with 20 column volumes of 10 mM KPi, pH 8.0, 20% glycerol, 2.0 mM DTT, 1 mM EDTA, wash with the above buffer with 75 mM KCl in order to remove any trace of detergent, then eluted with the above buffer with KCl concentration increased to 500 mM.

The protein was concentrated up to 40 mg/ml using a microconcentrator for crystallization assays.

### Protein Characterization

The quality of the final preparation was evaluated by:
*(a) SDS polyacrylamide gel electrophoresis* was performed using commercial gels (Nugen) followed by CBB staining according to the manufacturer's instructions. The purity as estimated by scanning a digital image of a gel was estimated to be at least 95%.
*(b) Mass Spectroscopy*
Mass spectroscopy was performed using a Bruker "BioTOF" electrospray time of flight instrument. Samples were either diluted by a factor of 1000 straight from storage buffer into methanol/water/formic acid (50:48:2 v/v/v), or subjected to reverse phase HPLC separation using a C4 column.
Calibration was achieved using Bombesin and angiotensin I using the 2+ and 1+ charged states. Data were acquired between 200 and 2000m/z range and were subsequently processed using Bruker's X-mass program. Mass accuracy was typically below 1 in 10 000.

| | |
|---|---|
| Mass spec of 2D6: | 53606.8 Da (observed) |
| | 53604.0 Da (predicted for minus N-terminal methionine) |

### Crystallization of 2D6

Crystals of the 2D6 were grown using the hanging drop vapor diffusion method. Protein at 40 mg/ml in 10mM Kpi pH 7.4, 0.5 M KC1, 2mM DTT, 1mM EDTA. 20% glycerol, was mixed in a 1:1 ratio, using 0.5ul drops, with a reservoir solution. The crystals of 2D6 grew over a reservoir solution containing:
0.1-0.15 M tri-sodium citrate dihydrate pH5.6, 5-10% Isopropanol (IPA), 5-15 % Peg 4000, 0-5% PEG 400.

Preferred conditions are:
0.15 M Tri-Sodium Citrate Dihydrate pH 5.6, 5% 2-propanol 5% PEG 4000
0.15 M Tri-Sodium Citrate Dihydrate pH 5.6, 5% 2-propanol 10% PEG 4000
0.15 M Tri-Sodium Citrate Dihydrate pH 5.6, 10% Iso-Propanol 5% PEG 4000
0.15 M Tri-Sodium Citrate Dihydrate pH 5.6, 10% Iso-Propanol, 7.5% PEG 4000
0.1 M tri-sodium citrate pH5.6, 10% IPA, 10% PEG 4000
0.15 M tri-sodium citrate pH5.6, 10% IPA, 10% PEG 4000
0.15 M tri-sodium citrate pH5.6, 10% IPA, 15% PEG 4000
0.15 M tri-sodium citrate pH5.6, 5% IPA, 7.5% PEG 4000
0.15 M Tri-Sodium Citrate Dihydrate pH 5.6, 10% Iso-Propanol,
7.5% PEG 4000, 5% PEG 400

Also,
0.1 M Tri-Sodium Citrate Dihydrate pH 5.6, 10% Iso-Propanol 7.5% PEG 4000
0.1 M Tri-Sodium Citrate Dihydrate pH 5.6, 10% Iso-Propanol 5% PEG 4000
0.15 M Tri-Sodium Citrate Dihydrate pH 5.6, 10% 2-propanol, 7.5% peg 8000
0.15 M Tri-Sodium Citrate Dihydrate 5.6, 10% 2-propanol, 5% glycerol, 7.5% peg 4000
0.1 M HEPES pH 7.5, 0.2 M Magnesium chloride , 30% PEG 400
0.1 M imidazole pH 8, 0.2 M calcium acetate, 10% PEG 8000
0.1 M Sodium Cacodylate pH 6.5, 0.2 M Magnesium Acetate, 15% 2-methyl-2,4-pentanediol
0.1 M Tris-HCl pH 8.5, 0 .2 M sodium acetate, 15% PEG 4000 0.150 Bis-trispropane pH 5.8, 10% 2-propanol , 7.5% PEG 4K
0.15 M tri-sodium citrate pH5.6, 10% isoproponal, 5% PEG 4000 with additives such as 10 mM strontium chloride, 10 mM yttrium chloride, 10 mM magnesium chloride, 10 mM manganese chloride, 3% ethanol, 10 mM taurine, 3% d-sucrose, 4% n-propanol, 4% 1,3butanediol etc

Crystals formed within 1-7 days at 25 °C, as irregular plates.

The crystals were flash frozen in liquid nitrogen, using 80% reservoir solution, 20% ethylene glycol as a cryoprotectant.

### Example 4 - Purification and Crystallisation of 3A4:

An N-terminal truncation used for 3A4 was the NF10 N-terminal truncation which results in an N-terminal sequence of MAYGTHSHGLFKK (SEQ ID NO:11) described by Gillam et al, Arch. Biochem. Biophys. Vol. 305, 123-131, 1993. A four histidine tag was inserted at the 3' terminus to facilitate the purification of the proteins in high salt buffers.

### Cloning of 3A4

3A4 corresponding to M18907 (GI_181373) was cloned from human liver library (Origene Technologies, Inc.).
PCR carried out as recommended by the manufacturer:

| | |
|---|---|
| Liver library | 2.0µl |
| 10X PCR buffer (-Mg²⁺) | 2.5µl |
| 10mM dNTPs | 0.5 µl |
| 10mM MgSO₄ | 2.5µl |
| Water | 11.0µl |
| Primer 1 (@10pmol/µl) | 3.0µl |
| Primer 2 (@10pmol/µl) | 3.0µl |

Primer 1 is complementary to the 5' end of the full length 3A4 cDNA. Primer 2 is complementary to the 3' end of the cDNA and adds a tetra-his tag onto the C-terminus of the 3A4 protein.

| Heat to 94°C, add 0.5µl (1 Unit) Vent polymerase 35 cycles as follows: | |
|---|---|
| 94°C | 30 seconds |
| 65°C | 60 seconds |
| 72°C | 60 seconds |

1 cycle of 72°C for 5 minutes.

Following the addition of 1µl (2.5 Units) Taq polymerase and incubation at 72°C for 10 minutes, 1µl of product was used in a TOPO cloning reaction (vector pCR4TOPO). The cloning reaction was used to transform *E. coli* XL1-blue and positive clones identified by NdeI/SalI restriction digestion of purified plasmids. Positive clones were sequenced fully and the NdeI/SalI insert subcloned into pET20b to yield clone 1384. This clone was used as the template in subsequent pcr reactions.

### N-Terminal truncation of 3A4

N-terminal truncation of 3A4 to generate the published NF10 N-terminal truncation described in Gillam et al, Arch. Biochem. Biophys. Vol. 305, 123-131, 1993.

| | |
|---|---|
| Template (1384) | ~5ng |
| 10X PCR buffer (+Mg²⁺) | 5.0µl |
| 10mM dNTPs | 1.0µl |
| Water | 42.0µl |
| Primer 2 (@100pmol/µl) | 0.5µl |
| Primer 3 (@100pmol/µl) | 0.5µl |
| Vent polymerase (2U/µl) | 0.5µl |

| 25 cycles of: | |
|---|---|
| 94°C | 30 seconds |
| 65°C | 60 seconds |
| 72°C | 60 seconds |

1 cycle of 72°C for 5 minutes.

Following the addition of 1µl (2.5 units) Taq polymerase and incubation at 72°C for 10 minutes, 1µl of product was used in a TOPO cloning reaction (vector pCR4TOPO). The cloning reaction was used to transform E. *coli* XL1-blue and positive clones identified by NdeI/SalI restriction digestion of purified plasmids. Positive clones were sequenced fully and the NdeI/SalI insert subcloned into pCWori+ to yield clone p3A4 having the coding sequence shown in SEQ ID NO:7. This clone was used for protein expression of the protein of SEQ ID NO:8.

Primer 1
5′-GGAATTCATATGGCTCTCATCCCAGACTTGGCC-3′ (SEQ ID NO:74)

Primer 2
5′-TGCGGTCGACTCAATGGTGATGGTGGGCTCCACTTACGGTGCCATCC-3′ (SEQ ID
NO:75)

Primer 3

5′-TTAACATATGGCATATGGTACTCATTCACATGGTCTGTTTAAAAAACT

GGGAATTCCAGGGCCCACACC-3′ (SEQ ID NO:76)

### Bacteria expression

A single ampicillin resistant colony of XL1 blue cells was grown overnight at 37 °C in Terrific Broth (TB) with shaking to near saturation and used to inoculate fresh TB media. Bacteria were grown to an OD600nm =0.5 in 1 liter of TB broth containing 100 µg/ml of ampicillin at 37°C at 185 rpm in 2 liter flask. The heme precursor delta aminolevulinic acid (80 mg/l) was added 30 min prior to induction with 1 mM isopropyl-(β-D-thiogalactopyranoside (IPTG) and the temperature lowered to 25 °C. The bacterial culture was continued under agitation at 25 °C for 48 hours.

### Protein purification

The cells were pelleted at 10000 g for 10 min and resuspended in a buffer containing 500 mM KPi, pH 7.4, 20 % glycerol, 10 mM mercaptoethanol, 0.1% (v/v) of protease inhibitor cocktail (Calbiochem), 10 mM imidazole, 40U/ml DNase 1 and 5 mM MgSO₄.

The cells were lysed by passing twice through a Constant Systems Cell Homogeniser at 10000 psi. The cell debris was then removed by centrifugation at 22000 x g at 4 °C for 30 min.

Detergent IGEPAL CA630 (Sigma) was added dropwise from a 10% stock solution to the lysate at a final concentration of 0.3% (v/v) and the lysate was incubated with previously washed NiNTA resin (Qiagen) overnight at 4 °C, using agitation. The protein bound-NiNTA resin was pelleted by centrifugation at 2000 g for 2 min at 4 °C. The resin was washed with 20 resin volumes of 500 mM KPi, pH 7.4, 20% glycerol, 10 mM mercaptoethanol, 10 mM imidazole, 1:1000 dilution of protease inhibitor cocktail, 0.3%(v/v) IGEPAL CA630 and the resin pelleted by centrifugation at 2000 xg for 2 min at 4 °C. The resin was then washed with 10 resin volumes of 500 mM KPi, pH 7.4, 20% glycerol, 10 mM mercaptoethanol, 20 mM imidazole, 0.1% (v/v) protease inhibitors, 0.3% IGEPAL CA630 and the resin recovered by centrifugation as described above.

The resin was packed into a column at 4 °C and the cytochrome P450 eluted with 500 mM KPi, pH 7.4, 20 % glycerol, 10 mM mercaptoethanol, 300 mM imidazole, 0.1% (v/v) of protease inhibitor cocktail, 0.3%(v/v) IGEPAL CA630.

The cytochrome P450 obtained from the NiNTA column was quickly desalted into 10 mM KPi, pH 7.4, 20% glycerol, 2.0 mM DTT, 1 mM EDTA using a HiPrep 26/10 desalting column (Pharmacia), at a flow rate of 5 ml/min.

The desalted cytochrome P450 was directly applied to a CM Sepharose column (Pharmacia), previously equilibrated with 10 mM KPi, pH 7.4, 20% glycerol, 2.0 mM DTT, 1 mM EDTA. The following step elution was applied: wash with 20 column volumes of 10 mM KPi, pH 7.4, 20% glycerol, 2.0 mM DTT, 1 mM EDTA, wash with the above buffer with 75 mM KCl in order to remove any trace of detergent, then eluted with the above buffer with KCl concentration increased to 500 mM.

The protein was concentrated up to 40 mg/ml using a microconcentrator for crystallization assays.

### Protein Characterization

The quality of the final preparation was evaluated by:
*(a) SDS polyacrylamide gel electrophoresis* was performed using commercial gels (Nugen) followed by CBB staining according to the manufacturer's instructions. The purity as estimated by scanning a digital image of a gel was estimated to be at least 95%.
*(b) Mass Spectroscopy* Mass spectroscopy was performed using a Bruker "BioTOF" electrospray time of flight instrument. Samples were either diluted by a factor of 1000 straight from storage buffer into methanol/water/formic acid (50:48:2 v/v/v), or subjected to reverse phase HPLC separation using a C4 column.
Calibration was achieved using Bombesin and angiotensin I using the 2+ and 1+ charged states. Data were acquired between 200 and 2000*m*/*z* range and were subsequently processed using Bruker's X-mass program. Mass accuracy was typically below 1 in 10 000.

| | |
|---|---|
| Mass spec of 3A4: | 55281 Da(observed) |
| | 55278 Da (predicted minus N-terminal methionine) |

### Crystallization of 3A4

Crystals of the 3A4 were grown using the hanging drop vapor diffusion method. Protein at 40 mg/ml in 10mM Kpi pH 7.4, 0.5 M KCl, 2mM DTT, 1mM EDTA. 20% glycerol, was mixed in a 1:1 ratio, using 0.5ul drops, with a reservoir solution. The crystals of 3A4 grew over a reservoir solution containing 0.1 M HEPES pH 7.5, 0.2 M sodium chloride, 30% PEG 400.

Alterative conditions are listed below:
0.1 M HEPES pH 7.5, 0.2 M sodium chloride, 30% PEG 400
0.05 M HEPES pH 7.5, 0.2 M sodium chloride, 35% PEG 400
0.05 M HEPES pH 7.5, 0.2 M sodium chloride, 30% PEG 400
0.15 M Imidazole-HCl pH 8, 10% 2-propanol
0.1 M 2-(N-cyclohexylamino)ethanesulfonic acid (CHES) pH 9.5, 30% PEG 400
0.15 M Hepes - Na pH 7.5, 5% IPA, 10% Peg 4000
0.1 M phosphate-citrate pH 4.2, 1.6 M NaH2PO4/ 0.4M K2HPO4
0.1 M citrate pH 5.5, 0.2 sodium chloride, 1.0 M Ammonium phosphate
0.2 M Lithium chloride, 20% PEG 3350
0.2 M Potassium chloride , 20% PEG 3350
0.2 M Sodium formate , 20% PEG 3350
0.2 M Potassium formate , 20% PEG 3350
0.2 M Ammonium formate , 20% PEG 3350
0.2 M Lithium acetate, 20% PEG 3350
0.2 M Potassium chloride, 20% PEG 3350
0.2 M Sodium formate , 20% PEG 3350
0.2 M Lithium acetate , 20% PEG 3350
0.2 M Sodium acetate , 20% PEG 3350
0.2 M Potassium acetate , 20% PEG 3350
0.2 M Ammonium acetate, 20% PEG 3350
0 .1 M HEPES pH 7.5, 0.2 M sodium chloride, 30% PEG 400
0.1 M HEPES pH 7.5, 5% Iso-Propanol, 10% PEG 4000
200 mM K Acetate, 25% peg 3350
200 mM K Acetate , 25% peg 3350
300 mM Na acetate, 25% peg 3350
200 mM Sodium formate, 25% PEG 3350
0. 300 M Lithium acetate, 25.0 % PEG 3350
0. 100 M Imidazole-HCl pH 8, 10% 2-propanol
0. 150 M Imidazole-HCl pH 8, 10% 2-propanol
Crystals formed within 1-7 days at 25 °C, and were rod shaped in morphology.

The approximate cell dimensions of the crystals were a=77 Å, b=99 Å, c=129 Å, β=90 °. The space group is I222. The invention thus provides crystal of 3A4 having this space group and unit cell dimensions, the dimensions a, b and c varying independently by +/- 5%.

The crystals were flash frozen in liquid nitrogen, using 80% reservoir solution, 20% ethylene glycol as a cryoprotectant.

Crystals of 3A4 were also grown over a reservoir solution containing:
0.15M HEPES pH7.5, 5% IPA, 10 % PEG 4000.

Crystals were obtained with unit cell C2: a=152Å, b=101 Å, c=78Å, α=90°, β=120°, γ=90°. The invention thus provides crystal of 3A4 having this space group and unit cell dimensions, the dimensions a, b and c and β varying independently by +/- 5%.

SEQUENCE LISTING

SEQ ID NO:1

2C19 (internal deletion, and His tagged) coding sequence.

ATGGCTAAGAAAACGAGCTCTAAAGGGCGGCCGCCTGGCCCCACTCCTCTCCCAGTGATTGGAAATATCCTACAGATAGA
TATTAAGGATGTCAGCAAATCCTTAACCAATCTCTCAAAAATCTATGGCCCTGTGTTCACTCTGTATTTTGGCCTGGAAC
GCATGGTGGTGCTGCATGGATATGAAGTGGTGAAGGAAGCCCTGATTGATCTTGGAGAGGAGTTTTCTGGAAGAGGCCAT
TTCCCACTGGCTGAAAGAGCTAACAGAGGATTTGGAATCGTTTTCAGCAATGGAAAGAGATGGAAGGAGATCCGGCGTTT
CTCCCTCATGACGCTGCGGAATTTTGGGATGGGGAAGAGGAGCATTGAGGACCGTGTTCAAGAGGAAGCCCACTGCCTTG
TGGAGGAGTTGAGAAAAACCAAGGCTTCACCCTGTGATCCCACTTTCATCCTGGGCTGTGCTCCCTGCAATGTGATCTGC
TCCATTATTTTCCAGAAACGTTTCGATTATAAAGATCAGCAATTTCTTAACTTGATGGAAAAATTGAATGAAAACATCAG
GATTGTAAGCACCCCCTGGATCCAGATATGCAATAATTTTCCCACTATCATTGATTATTTCCCGGGAACCCATAACAAAT
TACTTAAAAACCTTGCTTTTATGGAAAGTGATATTTTGGAGAAAGTAAAAGAACACCAAGAATCGATGGACATCAACAAC
CCTCGGGACTTTATTGATTGCTTCCTGATCAAAATGGAGAAGGAAAAGCAAAACCAACAGTCTGAATTCACTATTGAAAA
CTTGGTAATCACTGCAGCTGACTTACTTGGAGCTGGGACAGAGACAACAAGCACAACCCTGAGATATGCTCTCCTTCTCC
TGCTGAAGCACCCAGAGGTCACAGCTAAAGTCCAGGAAGAGATTGAACGTGTCGTTGGCAGAAACCGGAGCCCCTGCATG
CAGGACAGGGGCCACATGCCCTACACAGATGCTGTGGTGCACGAGGTCCAGAGATACATCGACCTCATCCCCACCAGCCT
GCCCCATGCAGTGACCTGTGACGTTAAATTCAGAAACTACCTCATTCCCAAGGGCACAACCATATTAACTTCCCTCACTT
CTGTGCTACATGACAACAAAGAATTTCCCAACCCAGAGATGTTTGACCCTCGTCACTTTCTGCATGAAGGTGGAAATTTT
AAGAAAAGTAACTACTTCATGCCTTTCTCAGCAGGAAAACGGATTTGTGTGGGAGAGGGCCTGGCCCGCATGGAGCTGTT
TTTATTCCTGACCTTCATTTTACAGAACTTTAACCTGAAATCTCTGATTGACCCAAAGGACCTTGACACAACTCCTGTTG
TCAATGGATTTGCTTCTGTCCCGCCCTTCTACCAGCTCTGCTTCATTCCTGTCCACCACCACCACTGA

SEQ ID NO:2

Protein sequence of 2C19 coded by SEQ ID NO:1

MAKKTSSKGRPPGPTPLPVIGNILQIDIKDVSKSLTNLSKIYGPVFTLYFGLERMVVLHGYEVVKEALIDLGEEF
SGRGHFPLAERANRGFGIVFSNGKRWKEIRRFSLMTLRNFGMGKRSIEDRVQEEAHCLVEELRKTKASPCDPTFI
LGCAPCNVICSIIFQKRFDYKDQQFLNLMEKLNENIRIVSTPWIQICNNFPTIIDYFPGTHNKLLKNLAFMESDI
LEKVKEHQESMDINNPRDFIDCFLIKMEKEKQNQQSEFTIENLVITAADLLGAGTETTSTTLRYALLLLLKHPEV
TAKVQEEIERVVGRNRSPCMQDRGHMPYTDAVVHEVQRYIDLIPTSLPHAVTCDVKFRNYLIPKGTTILTSLTSV
LHDNKEFPNPEMFDPRHFLHEGGNFKKSNYFMPFSAGKRICVGEGLARMELFLFLTFILQNFNLKSLIDPKDLDT
TPVVNGFASVPPFYQLCFIPVHHHH

SEQ ID No:3
2C19 wild type 1B.

ATGGCTAAGAAAACGAGCTCTAAAGGGCGGCCGCCTGGCCCTACTCCTCTCCCAGTGATTGGAAATATCCTACAG
ATAGATATTAAGGATGTCAGCAAATCCTTAACCAATCTCTCAAAAATCTATGGCCCTGTGTTCACTCTGTATTTT
GGCCTGGAACGCATGGTGGTGCTGCATGGATATGAAGTGGTGAAGGAAGCCCTGATTGATCTTGGAGAGGAGTTT
TCTGGAAGAGGCCATTTCCCACTGGCTGAAAGAGCTAACAGAGGATTTGGAATCGTTTTCAGCAATGGAAAGAGA
TGGAAGGAGATCCGGCGTTTCTCCCTCATGACGCTGCGGAATTTTGGGATGGGGAAGAGGAGCATTGAGGACCGT
GTTCAAGAGGAAGCCCGCTGCCTTGTGGAGGAGTTGAGAAAAACCAAGGCTTCACCCTGTGATCCCACTTTCATC
CTGGGCTGTGCTCCCTGCAATGTGATCTGCTCCATTATTTTCCAGAAACGTTTCGATTATAAAGATCAGCAATTT
CTTAACTTGATGGAAAAATTGAATGAAAACATCAGGATTGTAAGCACCCCCTGGATCCAGATATGCAATAATTTT
CCCACTATCATTGATTATTTCCCGGGAACCCATAACAAATTACTTAAAAACCTTGCTTTTATGGAAAGTGATATT
TTGGAGAAAGTAAAAGAACACCAAGAATCGATGGACATCAACAACCCTCGGGACTTTATTGATTGCTTCCTGATC
AAAATGGAGAAGGAAAAGCAAAACCAACAGTCTGAATTCACTATTGAAAACTTGGTAATCACTGCAGCTGACTTA
CTTGGAGCTGGGACAGAGACAACAAGCACAACCCTGAGATATGCTCTCCTTCTCCTGCTGAAGCACCCAGAGGTC
ACAGCTAAAGTCCAGGAAGAGATTGAACGTGTCGTTGGCAGAAACCGGAGCCCCTGCATGCAGGACAGGGGCCAC
ATGCCCTACACAGATGCTGTGGTGCACGAGGTCCAGAGATACATCGACCTCATCCCCACCAGCCTGCCCCATGCA
GTGACCTGTGACGTTAAATTCAGAAACTACCTCATTCCCAAGGGCACAACCATATTAACTTCCCTCACTTCTGTG
CTACATGACAACAAAGAATTTCCCAACCCAGAGATGTTTGACCCTCGTCACTTTCTGGATGAAGGTGGAAATTTT
AAGAAAAGTAACTACTTCATGCCTTTCTCAGCAGGAAAACGGATTTGTGTGGGAGAGGGCCTGGCCCGCATGGAG

CTGTTTTTATTCCTGACCTTCATTTTACAGAACTTTAACCTGAAATCTCTGATTGACCCAAAGGACCTTGACACA
ACTCCTGTTGTCAATGGATTTGCTTCTGTCCCGCCCTTCTACCAGCTCTGCTTCATTCCTGTCCACCACCACCAC
TGA

SEQ ID NO:4
Translation of SEQ ID NO:3

MAKKTSSKGRPPGPTPLPVIGNILQIDIKDVSKSLTNLSKIYGPVFTLYFGLERMVVLHGYEVVKEALIDLGEEF
SGRGHFPLAERANRGFGIVFSNGKRWKEIRRFSLMTLRNFGMGKRSIEDRVQEEARCLVEELRKTKASPCDPTFI
LGCAPCNVICSIIFQKRFDYKDQQFLNLMEKLNENIRIVSTPWIQICNNFPTIIDYFPGTHNKLLKNLAFMESDI
LEKVKEHQESMDINNPRDFIDCFLIKMEKEKQNQQSEFTIENLVITAADLLGAGTETTSTTLRYALLLLLKHPEV
TAKVQEEIERVVGRNRSPCMQDRGHMPYTDAVVHEVQRYIDLIPTSLPHAVTCDVKFRNYLIPKGTTILTSLTSV
LHDNKEFPNPEMFDPRHFLDEGGNFKKSNYFMPFSAGKRICVGEGLARMELFLFLTFILQNFNLKSLIDPKDLDT
TPVVNGFASVPPFYQLCFIPVHHHH

SEQ ID No:5
2D6 encoding nucleic acid

ATGGCTAAAAAAACCTCTTCTAAAGGCCGACCGCCGGGTCCGCTGCCGCTGCCAGGCCTGGGTAACCTGCTGCAT
GTGGACTTCCAGAACACCCCGTACTGCTTCGACCAGCTGCGTCGTCGTTTCGGTGACGTGTTCTCTCTGCAGCTG
GCTTGGACCCCGGTTGTTGTTCTGAACGGTCTGGCTGCTGTTCGCGAAGCTCTGGTTACCCACGGTGAAGACACC
GCTGACCGTCCGCCGGTCCCGATCACCCAGATCCTGGGTTTTGGTCCGCGTTCCCAAGGTGTTTTCCTGGCTCGT
TACGGACCGGCTTGGCGTGAACAGCGTCGTTTCTCTGTTTCTACCCTGCGTAACCTGGGTCTGGGTAAAAAATCT
CTGGAACAGTGGGTTACCGAAGAAGCTGCATGCCTGTGCGCTGCTTTCGCTAACCACTCTGGTCGTCCGTTCCGT
CCGAACGGTCTGCTGGACAAAGCTGTTTCTAACGTTATCGCTTCTCTGACCTGCGGCCGCCGTTTCGAATACGAC
GACCCGCGTTTCCTGCGTCTGCTGGACCTGGCTCAGGAAGGTCTGAAAGAGGAGTCTGGTTTCCTGCGTGAAGTT
CTGAACGCTGTTCCGGTTCTGCTGCACATCCCAGCTCTGGCTGGTAAAGTTCTGCGTTTCCAGAAAGCATTCCTG
ACCCAGCTGGACGAACTGCTGACCGAACACCGTATGACCTGGGACCCGGCTCAGCCGCCACGTGACCTGACCGAA
GCTTTCCTGGCTGAAATGGAAAAAGCTAAAGGTAACCCGGAATCTTCTTTCAACGATGAAAATCTGCGTATCGTT
GTTGCTGACCTGTTCTCCGCGGGTATGGTTACCACCTCTACCACCCTGGCTTGGGGTCTGCTGCTGATGATCCTG
CACCCGGATGTACAGCGTCGTGTTCAGCAGGAAATCGACGACGTTATTGGCCAGGTTCGTCGGCCGGAAATGGGT
GACCAGGCTCACATGCCGTACACCACCGCTGTTATCCACGAAGTTCAGCGCTTCGGTGACATCGTTCCGCTGGGT
ATGACCCACATGACCTCTCGTGACATCGAAGTTCAGGGTTTCCGTATCCCGAAAGGTACCACCCTGATCACCAAC
CTGTCTTCTGTTCTGAAAGACGAAGCTGTTTGGGAAAAACCGTTCCGTTTCCATCCGGAACACTTCCTGGACGCT
CAGGGTCACTTCGTTAAACCGGAAGCTTTCCTGCCGTTCTCTGCTGGTCGTCGTGCTTGCCTGGGTGAACCGCTG
GCTCGTATGGAACTGTTCCTGTTCTTCACCTCTCTGCTGCAGCACTTCTCTTTCTCTGTTCCGACCGGTCAGCCG
CGTCCGTCTCACCACGGTGTTTTCGCTTTCCTGGTTTCTCCGTCTCCGTACGAACTGTGCGCTGTTCCGCGTGGA
GCTCACCACCACCACTGA

SEQ ID NO 6:
Translation of SEQ ID NO:5

MAKKTSSKGRPPGPLPLPGLGNLLHVDFQNTPYCFDQLRRRFGDVFSLQLAWTPVVVLNGLAAVREALVTHGEDT
ADRPPVPITQILGFGPRSQGVFLARYGPAWREQRRFSVSTLRNLGLGKKSLEQWVTEEAACLCAAFANHSGRPFR
PNGLLDKAVSNVIASLTCGRRFEYDDPRFLRLLDLAQEGLKEESGFLREVLNAVPVLLHIPALAGKVLRFQKAFL
TQLDELLTEHRMTWDPAQPPRDLTEAFLAEMEKAKGNPESSFNDENLRIVVADLFSAGMVTTSTTLAWGLLLMIL
HPDVQRRVQQEIDDVIGQVRRPEMGDQAHMPYTTAVIHEVQRFGDIVPLGMTHMTSRDIEVQGFRIPKGTTLITN
LSSVLKDEAVWEKPFRFHPEHFLDAQGHFVKPEAFLPFSAGRRACLGEPLARMELFLFFTSLLQHFSFSVPTGQP
RPSHHGVFAFLVSPSPYELCAVPRGAHHHH

SEQ ID NO:7
3A4

ATGGCATACGGTACTCATTCACATGGTCTGTTTAAAAAACTGGGAATTCCAGGGCCCACACCTCTGCCTTTTTTG
GGAAATATATTTTGTCCTACCATAAGGGCTTTTGTATGTTTGACATGGAATGTCATAAAAAGTATGGAAAAGTGTGG
GGCTTTTATGATGGTCAACAGCCTGTGCTGGCTATCACAGATCCTGACATGATCAAAACAGTGCTAGTGAAAGAA
TGTTATTCTGTCTTCACAAACCGGAGGCCTTTTGGTCCAGTGGGATTTATGAAAAGTGCCATCTCTATAGCTGAG
GATGAAGAATGGAAGAGATTACGATCATTGCTGTCTCCAACCTTCACCAGTGGAAAACTCAAGGAGATGGTCCCT
ATCATTGCCCAGTATGGAGATGTGTTGGTGAGAAATCTGAGGCGGGAAGCAGAGACAGGCAAGCCTGTCACCTTG

AAAGACGTCTTTGGGGCCTACAGCATGGATGTGATCACTAGCACATCATTTGGAGTGAACATCGACTCTCTCAAC
AATCCACAAGACCCCTTTGTGGAAAACACCAAGAAGCTTTTAAGATTTGATTTTTTGGATCCATTCTTTCTCTCA
ATAACAGTCTTTCCATTCCTCATCCCAATTCTTGAAGTATTAAATATCTGTGTGTTTCCAAGAGAAGTTACAAAT
TTTTTAAGAAAATCTGTAAAAAGGATGAAAGAAAGTCGCCTCGAAGATACACAAAAGCACCGAGTGGATTTCCTT
CAGCTGATGATTGACTCTCAGAATTCAAAAGAAACTGAGTCCCACAAAGCTCTGTCCGATCTGGAGCTCGTGGCC
CAATCAATTATCTTTATTTTTGCTGGCTATGAAACCACGAGCAGTGTTCTCTCCTTCATTATGTATGAACTGGCC
ACTCACCCTGATGTCCAGCAGAAACTGCAGGAGGAAATTGATGCAGTTTTACCCAATAAGGCACCACCCACCTAT
GATACTGTGCTACAGATGGAGTATCTTGACATGGTGGTGAATGAAACGCTCAGATTATTCCCAATTGCTATGAGA
CTTGAGAGGGTCTGCAAAAAAGATGTTGAGATCAATGGGATGTTCATTCCCAAAGGGGTGGTGGTGATGATTCCA
AGCTATGCTCTTCACCGTGACCCAAAGTACTGGACAGAGCCTGAGAAGTTCCTCCCTGAAAGATTCAGCAAGAAG
AACAAGGACAACATAGATCCTTACATATACACACCCTTTGGAAGTGGACCCAGAAACTGCATTGGCATGAGGTTT
GCTCTCATGAACATGAAACTTGCTCTAATCAGAGTCCTTCAGAACTTCTCCTTCAAACCTTGTAAAGAAACACAG
ATCCCCCTGAAATTAAGCTTAGGAGGACTTCTTCAACCAGAAAAACCCGTTGTTCTAAAGGTTGAGTCAAGGGAT
GGCACCGTAAGTGGAGCCCACCATCACCATTGA

SEQ ID NO:8
3A4

MAYGTHSHGLFKKLGIPGPTPLPFLGNILSYHKGFCMFDMECHKKYGKVWGFYDGQQPVLAITDPDMIKTVLVKE
CYSVFTNRRPFGPVGFMKSAISIAEDEEWKRLRSLLSPTFTSGKLKEMVPIIAQYGDVLVRNLRREAETGKPVTL
KDVFGAYSMDVITSTSFGVNIDSLNNPQDPFVENTKKLLRFDFLDPFFLSITVFPFLIPILEVLNICVFPREVTN
FLRKSVKRMKESRLEDTQKHRVDFLQLMIDSQNSKETESHKALSDLELVAQSIIFIFAGYETTSSVLSFIMYELA
THPDVQQKLQEEIDAVLPNKAPPTYDTVLQMEYLDMVVNETLRLFPIAMRLERVCKKDVEINGMFIPKGVVVMIP
SYALHRDPKYWTEPEKFLPERFSKKNKDNIDPYIYTPFGSGPRNCIGMRFALMNMKLALIRVLQNFSFKPCKETQ
IPLKLSLGGLLQPEKPVVLKVESRDGTVSGAHHHH

## Claims

1. A method for the purification of a cytochrome P450,
wherein said method comprises:
(a) expressing in a host cell culture a cytochrome P450 molecule;
(b) recovering said cells from said culture and suspending said cells in a salt buffer having a conductivity of from 12 to 110 mS/cm;
(c) lysing said cells and removing cell debris to provide a high-salt lysate;
(d) adding to said lysate a detergent to provide a high-salt-detergent lysate; and
(e) recovering said P450 from said lysate.

2. The method of claim 1 wherein the salt buffer has a salt concentration of from 200 to 1000 mM.

3. The method of claim 1 or 2 wherein the detergent is added at 0.015 to 1.2% v/v.

4. The method of any one of claims 1 to 3 wherein step (e) is performed by:
(e(i)) binding said P450 to an affinity support;
(e(ii)) rinsing said support in a high-salt-detergent wash;
(e(iii)) removing said P450 in a high-salt-detergent buffer to provide a P450-high-salt-detergent preparation; and
(f) rapidly desalting the preparation to provide a P450-low-salt preparation.

5. The method of claim 4 wherein step (f) is performed by removing salt from said preparation by size-exclusion chromatography.

6. The method of any one of the preceding claims wherein the P450 carries a polyhistidine tag.

7. The method of any one of the preceding claims wherein the P450 is a member of the CYP1, 2, 3 or 4 family.

8. The method of claim 7 wherein the P450 is a CYP2 family member.

9. The method of claim 8 wherein the P450 is 2C9 or 2C19.

10. The method of claim any one of the preceding claims wherein the P450 comprises a deletion in its N-terminal membrane inserting element.

11. The method of claim 10 wherein the N-terminal sequence of said P450 comprises, in place of the N-terminal membrane inserting element, a sequence MAKKTSSKGR or MAYGTHSHGLFKK.

12. The method of claim 11 wherein said P450 is of SEQ ID NO:2, 4, 6 or 8.

13. The method of any one of the preceding claims which further comprises crystallizing the P450.

14. A crystal of a cytochrome P450.

15. The crystal of claim 14 wherein said P450 is 2C19 and said crystal has cell dimensions of a=158Å, b=158Å, c=212Å (+/-5% for a, b, and c), α=90°, β=90°, γ=120°, and a space group P321.

16. The crystal of claim 14 wherein said P450 is 2D6.

17. The crystal of claim 14 wherein said P450 is 3A4 having a space group 1222 and unit cell size a=77 Å, b=99 Å, c=129 Å, (+/- 5% for a, b and c), β=90°; or having a space group C2 and unit cell size a=152Å, b=101 Å, c=78Å (+/- 5% for a, b and c), α=90°, β=120°, γ=90°.

18. A method for determining the crystal structure of a cytochrome P450 which comprises preparing a crystal according to the method of claim 13, subjecting the crystal to x-ray diffraction, and analysing the diffraction pattern obtained to determine the 3-dimensional coordinates of the atoms of said P450.

19. A nucleic acid for expression of cytochrome P450 2D6 having the coding sequence for 2D6 of SEQ ID NO:5.

20. A bacterial expression vector comprising the nucleic acid of claim 19.
